# EUROPEAN PATENT APPLICATION

(11) **EP 4 458 460 A1**
(43) Date of publication of application: **06.11.2024**
(21) Application number: 24173345.0
(22) Date of filing: 30.04.2024
(51) Int. Cl.: B01J 19/00, B01J 19/24, C07K 1/00

(54) **METHOD AND APPARATUS FOR PRODUCING PEPTIDE**

(30) Priority: 01.05.2023 JP 2023075533
(71) Applicant: Yokogawa Electric Corporation, Tokyo 180-8750 (JP)
(72) Inventor: Iwanaga, Natsumi, Musashino-shi, Tokyo, 180-8750 (JP); Otake, Yuma, Musashino-shi, Tokyo, 180-8750 (JP)
(74) Representative: Grünecker Patent- und Rechtsanwälte PartG mbB

(57) **Abstract**

The present invention provides a means for producing a peptide with a small number of steps in a short time. One aspect of the present invention relates to a method for producing a peptide, comprising: a C-terminal activation step of introducing an N-terminally protected amino acid or peptide, in which an N-terminal amino group is protected by an N-terminal protecting group, and a C-terminal activator into a flow reactor, thereby activating a C-terminal carboxyl group of the N-terminally protected amino acid or peptide in the flow reactor to form a C-terminally activated derivative of the N-terminally protected amino acid or peptide; a condensation step of introducing the C-terminally activated derivative of the N-terminally protected amino acid or peptide and a C-terminally protected amino acid or peptide, in which a C-terminal carboxyl group is protected by a tag protecting group, into a flow reactor, thereby condensing the N-terminally protected amino acid or peptide with the C-terminally protected amino acid or peptide on the N-terminal side in the flow reactor to form an N-terminally and C-terminally protected peptide extended N-terminally; an N-terminal deprotection step of introducing the N-terminally and C-terminally protected peptide extended N-terminally and a deprotecting agent for an N-terminal protecting group into a flow reactor, thereby removing the N-terminal protecting group for deprotection in the flow reactor to form a C-terminally protected peptide extended N-terminally; and an extraction step of mixing a reaction mixture containing the C-terminally protected peptide extended N-terminally with an aqueous phase to extract the C-terminally protected peptide extended N-terminally into an organic phase. Another aspect of the present invention relates to an apparatus for producing a peptide.

## Description

### CROSS REFERENCE TO RELATED APPLICATIONS

The present application claims priority from Japanese patent application JP 2023-075533 filed on May 1, 2023, the entire content of which is hereby incorporated by reference into this application.

### BACKGROUND

### Technical Field

The present invention relates to a method and apparatus for producing a peptide. In particular, the present invention relates to a method and apparatus for continuously producing a peptide using flow reactors.

### Background Art

Peptides have been widely used as components of, for example, pharmaceuticals and foods. Peptide synthesis has been under research and development for 100 years or more. Currently, liquid-phase and solid-phase methods are commonly used.

In the solid-phase method, the C-terminus of an amino acid or peptide is usually attached to a solid-phase support, followed by an extension reaction of a peptide chain on the N-terminal side. In the case of the solid-phase method, unreacted raw materials and reactants, as well as by-products, can be easily removed by washing the solid-phase support. On the other hand, reactivity is low in the solid-phase method since the extension reaction is performed on the solid-phase support, resulting in a time-consuming peptide production.

In the liquid-phase method, reactivity is high since the extension reaction of the peptide chain is performed in a reaction solution containing a raw material and a reactant, and therefore, the scale of production can be easily adjusted. However, since the liquid-phase method involves separation and purification of products for each condensation and deprotection reaction in a discontinuous process (batch processing), the production process becomes complicated, resulting in a time-consuming peptide production.

A flow synthesis method using flow reactors has been developed as a method to continuously carry out condensation and deprotection reactions in the liquid-phase method. The flow synthesis method is a method using microscopic flow channels having an inner diameter of a few millimeters or less as a reaction field (flow reactor). In the case of the flow synthesis method, the reaction time can be strictly controlled on the several second timescale since the reaction solution can be mixed instantaneously. In the case of the flow synthesis method, heating or cooling of a reaction system can be performed instantaneously since the specific surface area per unit volume of the reaction solution is remarkably large as compared with a general liquid-phase method of batch processing. Due to such characteristics, the flow synthesis method can reduce the time and energy cost required for the production of peptides (Otake, Y. et al., Angew. Chem. Int. Ed., 2020, Vol. 59, pp. 12925-12930; and Fuse, S. et al., Nature Communications, 2016, Vol. 7, p. 13491).

The flow synthesis method can reduce the time and energy cost required for extension reactions of peptide chains but requires separation and purification of products in batch processing for each condensation and deprotection reaction as in the liquid-phase method. In response to this problem in the liquid-phase method and the flow synthesis method, a protecting group capable of separating and purifying a product by two phase separation of an organic phase and an aqueous phase without solid-liquid separation as in the solid-phase method (hereinafter, also referred to as a "pseudo-solid-phase protecting group" or a "tag protecting group") has been developed.

For example, International Publication No. WO 2017/038650 describes a benzyl compound that may be used as such a protecting group.

International Publication No. WO 2020/218497 describes a method for producing a peptide using flow reactors and a pseudo-solid-phase protecting group.

### SUMMARY

As described above, a method for producing a peptide using flow reactors has been developed. However, there are several problems with the prior art methods. For example, in the case of the methods described in Otake, Y. et al., Angew. Chem. Int. Ed., 2020, Vol. 59, pp. 12925-12930; and Fuse, S. et al., Nature Communications, 2016, Vol. 7, p. 13491, it is necessary to carry out separation and purification of products in batch processing for each condensation and deprotection reaction, and therefore, there is a problem that it takes a long time to produce the peptide as a whole.

Accordingly, an object of the present invention is to provide a means for producing a peptide with a small number of steps in a short time.

The present inventors have studied various means for solving the problem. The present inventors have found that, in a method for producing a peptide using flow reactors, a deprotection reaction of an N-terminal protecting group can be continuously performed without purification of a peptide after a condensation reaction by using a tag protecting group capable of separating and purifying a product by two phase separation of an organic phase and an aqueous phase as a C-terminal protecting group, and a peptide can be thus produced in a short time. The present inventors have completed the present invention based on the above findings.

In other words, the present invention includes the following aspects and embodiments.

### (Embodiment 1)

A method for producing a peptide, comprising:
a C-terminal activation step of introducing an N-terminally protected amino acid or peptide, in which an N-terminal amino group is protected by an N-terminal protecting group, and a C-terminal activator into a flow reactor, thereby activating a C-terminal carboxyl group of the N-terminally protected amino acid or peptide in the flow reactor to form a C-terminally activated derivative of the N-terminally protected amino acid or peptide;
a condensation step of introducing the C-terminally activated derivative of the N-terminally protected amino acid or peptide and a C-terminally protected amino acid or peptide, in which a C-terminal carboxyl group is protected by a tag protecting group, into a flow reactor, thereby condensing the N-terminally protected amino acid or peptide with the C-terminally protected amino acid or peptide on the N-terminal side in the flow reactor to form an N-terminally and C-terminally protected peptide extended N-terminally;
an N-terminal deprotection step of introducing the N-terminally and C-terminally protected peptide extended N-terminally and a deprotecting agent for an N-terminal protecting group into a flow reactor, thereby removing the N-terminal protecting group for deprotection in the flow reactor to form a C-terminally protected peptide extended N-terminally; and
an extraction step of mixing a reaction mixture containing the C-terminally protected peptide extended N-terminally with an aqueous phase to extract the C-terminally protected peptide extended N-terminally into an organic phase.

### (Embodiment 2)

The method according to Embodiment 1, wherein the C-terminal activator is selected from the group consisting of halogenated ethyl formate, halogenated isopropyl formate, halogenated isobutyl formate, a carboxylic acid halide, phosgene and a phosgene equivalent, a carbodiimide condensing agent, a phosphonium condensing agent, an uronium condensing agent, a halouronium condensing agent, an imidazole condensing agent, a triazine condensing agent, and a condensation additive.

### (Embodiment 3)

The method according to Embodiment 1 or 2, wherein the tag protecting group is a monovalent group derived from a compound selected from the group consisting of:
(i) a fluorene compound represented by formula (I): 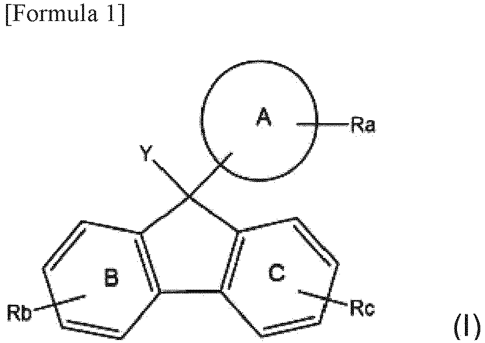 wherein
   Ring A represents an aromatic ring; Y is a hydroxyl group, a bromo group, or a chloro group; Ra, Rb, and Rc each independently represent an organic group having an aliphatic hydrocarbon group, a hydrogen atom, or an electron withdrawing group, and at least one of Ra, Rb, and Rc is an organic group having an aliphatic hydrocarbon group; and Rings A, B and C each independently optionally have an electron withdrawing group,
   wherein the organic group having an aliphatic hydrocarbon group is at least one group selected from the group consisting of
   a group represented by formula (b): 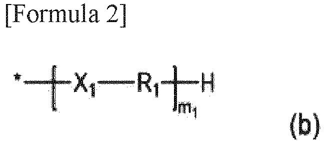 wherein
      * represents a binding position; X₁ is -O-; R₁ is an aliphatic hydrocarbon group having 5 to 60 carbon atoms; and m₁ is 1,
      a group represented by formula (c): 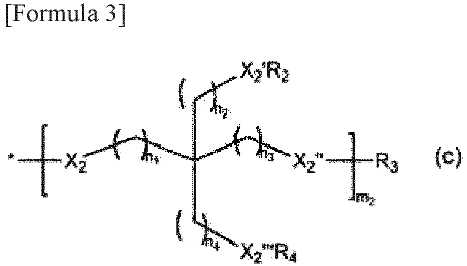 wherein
         * represents a binding position; X₂, X₂', X₂", and X₂‴ are -O-; R₂ and R₄ are each independently an aliphatic hydrocarbon group having 5 to 60 carbon atoms; R₃ is an organic group having an aliphatic hydrocarbon group having 5 to 60 carbon atoms; n₁, n₂, n₃, and n₄ are 1; and m₂ is 1, and
         a group represented by formula (d): 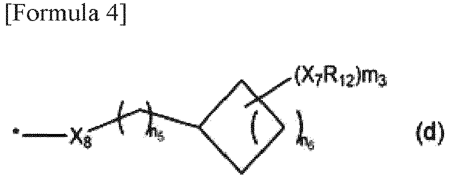 wherein
            * represents a binding position; X₈ represents -O-; m₃ is 2 or 3; n₅ is 1; n₆ is 3; X₇ is - O-; m₃ number of R₁₂ are each independently an alkyl group having 4 to 30 carbon atoms,
            wherein the organic group having an aliphatic hydrocarbon group is present at a 2- and/or 7-position of the fluorene compound,
(ii) a fluorene compound represented by formula (II): 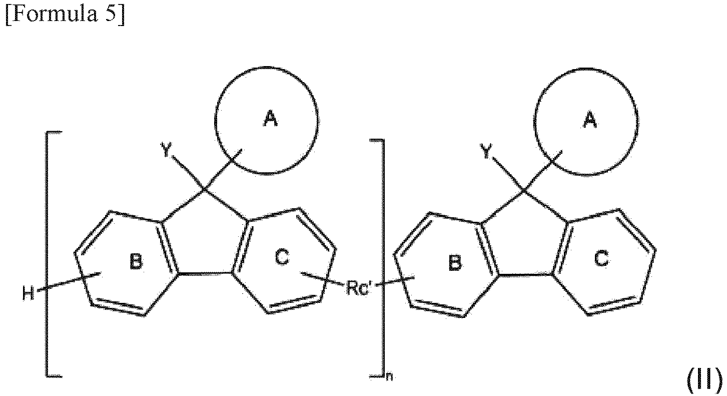 wherein
   Ring A represents an aromatic ring; Y is a hydroxyl group, a bromo group, or a chloro group; n represents an integer of 1 to 19; Rc' is a divalent organic group having an aliphatic hydrocarbon group; Rings A, B, and C each independently optionally have at least one selected from an organic group having an aliphatic hydrocarbon group and an electron withdrawing group; when a plurality of Rings A are present, Rings A are the same as or different from each other; when a plurality of Ys are present, Y are the same as or different from each other; and when a plurality of Rc' are present, Rc' are the same as or different from each other,
   wherein the divalent organic group having an aliphatic hydrocarbon group is a group represented by
   a group represented by formula (a): 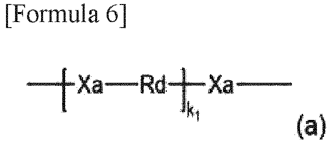 wherein
      Xa is absent or represents -O-, -S-, -NHCO-, or -CONH-; Rd represents an aliphatic hydrocarbon group having 5 or more carbon atoms; k₁ represents an integer of 1 to 10; when a plurality of Rd are present, Rd are the same as or different from each other; and when a plurality of Xa are present, respective Xa are the same as or different from each other, and
      wherein the organic group having an aliphatic hydrocarbon group is at least one group selected from the group consisting of
      a group represented by formula (b): 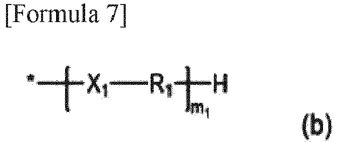 wherein
         * represents a binding position; X₁ is -O-; R₁ is an aliphatic hydrocarbon group having 5 to 60 carbon atoms; and m₁ is 1,
         a group represented by formula (c): 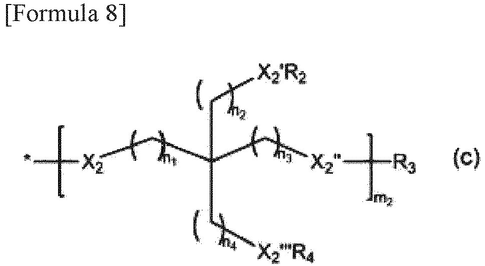 wherein
            * represents a binding position; X₂, X₂', X₂", and X₂‴ are -O-; R₂ and R₄ are each independently an aliphatic hydrocarbon group having 5 to 60 carbon atoms; R₃ is an organic group having an aliphatic hydrocarbon group having 5 to 60 carbon atoms; n₁, n₂, n₃, and n₄ are 1; and m₂ is 1, and
            a group represented by formula (d): 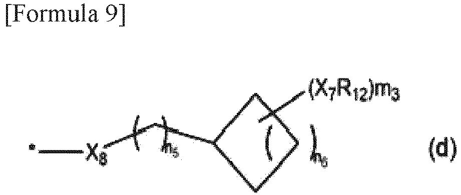 wherein
               * represents a binding position; X₈ represents -O-; m₃ is 2 or 3; n₅ is 1; n₆ is 3; X₇ is - O-; m₃ number of R₁₂ are each independently an alkyl group having 4 to 30 carbon atoms,
               wherein the organic group having the aliphatic hydrocarbon group is present at a 2- and/or 7-position of the fluorene compound,
(iii) a benzyl compound represented by formula (III): 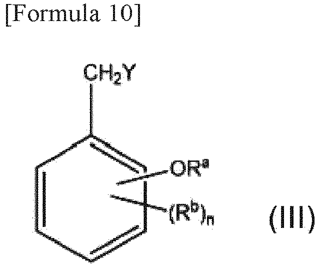 wherein
   Y represents a hydroxyl group or an -NHR group, where R represents a hydrogen atom, an alkyl group, or an aralkyl group;
   R^{a} is an organic group having an aliphatic hydrocarbon group selected from the group consisting of
   a group represented by formula (a): 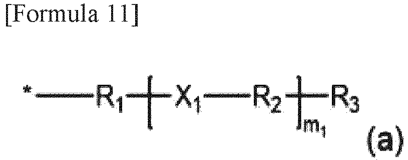 wherein
      * represents a binding position; m₁ represents an integer of 1 to 10; m₁ number of X₁ each independently represent a single bond or -O-, -S-, -COO-, -OCONH-, -NHCO-, or -CONH-; R₁ and m₁ number of R₂ each independently represent a divalent aliphatic hydrocarbon group having 5 or more carbon atoms; and R₃ is a hydrogen atom, or
         a group represented by formula (III'): 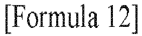 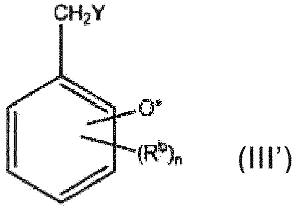 wherein
         Y represents a hydroxyl group or an -NHR group, where R represents a hydrogen atom, an alkyl group, or an aralkyl group; * represents a binding position; n number of R^{b} each independently represent an alkoxy group having 1 to 6 carbon atoms, a halogen atom, or an alkyl group having 1 to 6 carbon atoms optionally substituted with one or more halogen atoms; and n represents an integer of 0 to 4;
         a group represented by formula (b): 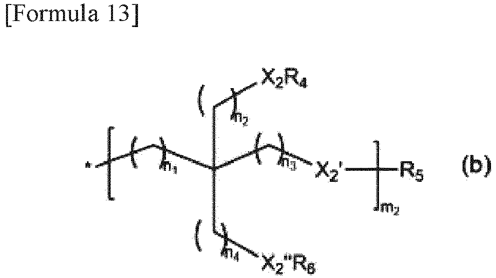 wherein
            * represents a binding position; m₂ represents 1 or 2; n₁, n₂, n₃, and n₄ each independently represent an integer of 0 to 2; m₂ number of X₂, m₂ number of X₂', and m₂ number of X₂" each independently represent a single bond or -O-, -S-, -COO-, -OCONH-, - NHCO-, or -CONH-; m₂ number of R₄ and m₂ number of R₆ each independently represent an aliphatic hydrocarbon group having 5 or more carbon atoms; and R₅ represents an aliphatic hydrocarbon group having 5 or more carbon atoms;
            a group represented by formula (c): 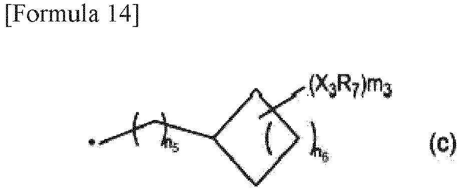 wherein
               * represents a binding position; m₃ represents an integer of 0 to 15; n₅ represents an integer of 0 to 11; n₆ represents an integer of 0 to 5; m₃ number of X₃ each independently represent a single bond or -O-, -S-, -COO-, -OCONH-, -NHCO-, or -CONH-; and m₃ number of R₇ each independently represent a hydrogen atom, a methyl group, or an aliphatic hydrocarbon group having 5 or more carbon atoms; and
               a group represented by formula (d): 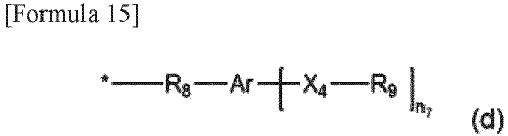 wherein
                  * represents a binding position; n₇ number of X₄ each independently represent a single bond or -O-, -S-, -COO-, -OCONH-, -NHCO-, or -CONH-; R₈ represents a divalent aliphatic hydrocarbon group; n₇ number of R₉ each independently represent a monovalent aliphatic hydrocarbon group; n₇ represents an integer of 1 to 5; and Ar represents an arylene group,
                  the organic group having a total number of carbon atoms of 30 or more;
                  n number of R^{b} each independently represent an alkoxy group having 1 to 6 carbon atoms, a halogen atom, or an alkyl group having 1 to 6 carbon atoms optionally substituted with one or more halogen atoms; and n represents an integer of 0 to 4,
(iv) a compound represented by formula (IV): 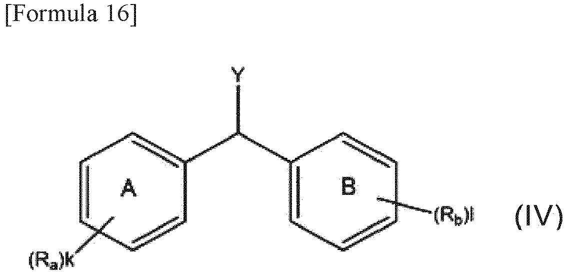 wherein
   Y represents a hydroxyl group or an -NHR group, where R represents a hydrogen atom, an alkyl group, or an aralkyl group; k and l each independently represent an integer of 0 to 5, provided that k+1 is not 0; k number of Rₐ and one R_{b} each independently represent an organic group having an aliphatic hydrocarbon group having 5 or more carbon atoms selected from the group consisting of
   a group represented by formula (a): 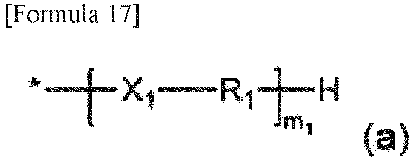 wherein
      * represents a binding position; m₁ represents an integer of 1 to 10; m₁ number of X₁ each independently are absent or represent -O-, -S-, -COO-, -OCONH-, or -CONH-; m₁ number of R₁ each independently represent a divalent aliphatic hydrocarbon group having 5 or more carbon atoms,
      a group represented by formula (b): 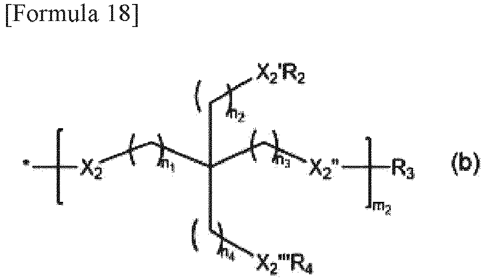 wherein
         * represents a binding position; m₂ represents an integer of 1 or 2; m₂ number of n₁, n₂, n₃, and n₄ each independently represent an integer of 0 to 2; m₂ number of X₂, m₂ number of X₂', m₂ number of X₂‴, and m₂ number of X₂" each independently are absent or represent -O-, -S-, -COO-, -OCONH-, or -CONH-; m₂ number of R₂ and R₄ each independently represent a hydrogen atom, a methyl group, or an aliphatic hydrocarbon group having 5 or more carbon atoms; and R₃ represents an aliphatic hydrocarbon group having 5 or more carbon atoms, and
         a group represented by formula (e): 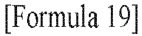 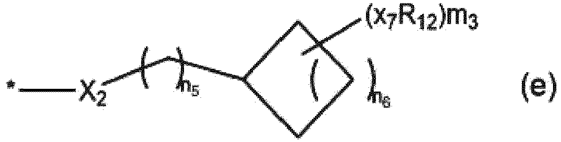 wherein
            * represents a binding position; m₃ represents an integer of 0 to 15; n₅ represents an integer of 0 to 11; n₆ represents an integer of 0 to 5; X₂ is absent or represents -O-, -S-, -NHCO-, or -CONH-; m₃ number of X₇ each independently are absent or represent -O-, -S-, -COO-, - OCONH-, -NHCO-, or -CONH-; and m₃ number of R₁₂ each independently represent a hydrogen atom, a methyl group, or an aliphatic hydrocarbon group having 5 or more carbon atoms,
            wherein the total number of carbon atoms of all aliphatic hydrocarbon groups in an organic group having k+1 number of aliphatic hydrocarbon groups is 16 or more; Ring A optionally further has a substituent in addition to Rₐ; and Ring B optionally further has a substituent in addition to R_{b},
(v) a branched chain-containing aromatic compound represented by formula (V): 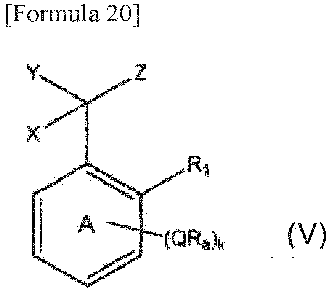 wherein
   k number of Q each independently represent a single bond or -O-, -S-, -C(=O)O-, - C(=O)NH-, or -NH-; k number of Rₐ each independently represent an organic group having at least one aliphatic hydrocarbon group having one or more branched chains with a total number of branched chains of 3 or more and a total number of carbon atoms of 14 or more and 300 or less; k represents an integer of 1 to 4; R₁ is a hydrogen atom or, when Z is a group represented by formula (a) below, represents a single bond together with R₂ to form a fluorene ring together with Ring B; Ring A optionally has at least one substituent selected from the group consisting of a halogen atom, a C1-6 alkyl group optionally substituted with one or more halogen atoms, and a C1-6 alkoxy group optionally substituted with one or more halogen atoms, in addition to R₁, k number of QRa, and C(X)(Y)Z; X represents a hydrogen atom or a phenyl group; Y represents a hydroxyl group or an -NHR group, where R represents a hydrogen atom, an alkyl group or an aralkyl group; and Z represents a hydrogen atom or
   a group represented by formula (a): 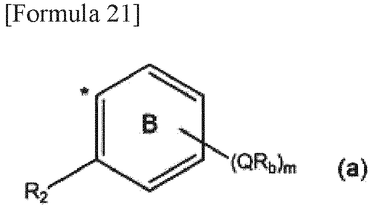 wherein
      * represents a binding position; m represents an integer of 0 to 4; m number of Q each independently represent a single bond or -O-, -S-, -C(=O)O-, -C(=O)NH-, or -NH-; m number of R_{b} each independently represent an organic group having at least one aliphatic hydrocarbon group having one or more branched chains with a total number of branched chains of 3 or more and a total number of carbon atoms of 14 or more and 300 or less; R₂ represents a hydrogen atom or represents a single bond together with R₁ to form a fluorene ring together with Ring A; and Ring B optionally has one or more substituents selected from the group consisting of a halogen atom, a C1-6 alkyl group optionally substituted with one or more halogen atoms, and a C1-6 alkoxy group optionally substituted with one or more halogen atoms, in addition to m QR_{b}s and R₂; and
      the organic group having at least one aliphatic hydrocarbon group having one or more branched chains with a total number of branched chains of 3 or more and a total number of carbon atoms of 14 or more and 300 or less in Rₐ and R_{b} is a group having 3 or more same or different divalent groups represented by
      a group represented by formula (b): 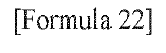 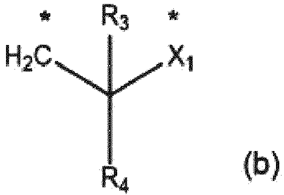 wherein
         * represents a binding position to an adjacent atom; R₃ and R₄ each independently represent a hydrogen atom or a C1-4 alkyl group; and X₁ represents a single bond, a C1-4 alkylene group, or an oxygen atom, provided that R₃ and R₄ are not simultaneously hydrogen atoms,
(vi) a compound represented by formula (VI): 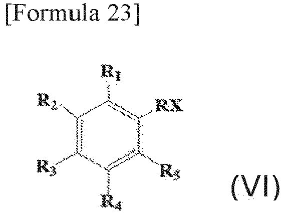 wherein
   R₁ and R₅ are each a hydrogen atom; R₂, R₃, and R₄ are each an alkoxy group having 8 to 30 carbon atoms; RX is a group binding to a carboxyl group, an amino group, or a hydroxy group of an amino acid, a peptide, or an amino acid amide or to a linker, which is represented by -CHO, -CH₂OH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group,
(vii) a compound represented by formula (VII): 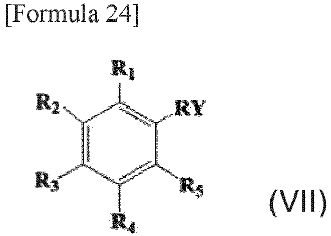 wherein
   R₂, R₄, and R₅ are each a hydrogen atom; R₁ and R₃ are each an alkoxy group having 12 to 30 carbon atoms; RY is a group binding to a carboxyl group, an amino group, or a hydroxy group of an amino acid, a peptide, or an amino acid amide or to a linker, which is represented by -CHO, -CH₂OH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group,
(viii) a compound represented by formula (VIII): 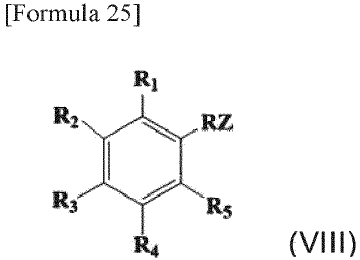 wherein
   R₁, R₃, and R₅ are each a hydrogen atom; R₂ and R₄ are each an alkoxy group having 12 to 30 carbon atoms; RZ is a group binding to a carboxyl group, an amino group, or a hydroxy group of an amino acid, a peptide, or an amino acid amide or to a linker, which is represented by -CHO, -CH₂OH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group, and
(ix) a compound represented by formula (IX): 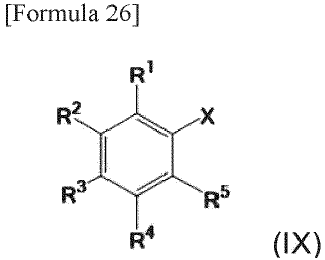 wherein
   X is a group representing -CH₂ORa where Ra represents a hydrogen atom, a halogenocarbonyl group, or an active ester protecting group, -CH₂NHRb where Rb represents a hydrogen atom, a linear or branched alkyl group having 1 to 6 carbon atoms, or an aralkyl group, a halogenomethyl group, a methyl azide group, a formyl group, or an oxime, and binding to a carboxyl group, an amino group, or a hydroxy group of an amino acid, a peptide, or an amino acid amide or to a linker; at least one of R¹, R², R³, R⁴ and R⁵ represents a group represented by a formula of -O-R⁶-Xa-A, and a residual group represents a hydrogen atom, a halogen atom, an alkyl group having 1 to 4 carbon atoms, or an alkoxy group having 1 to 4 carbon atoms; R⁶ represents a linear or branched alkylene group having 1 to 16 carbon atoms, and Xa represents O or CONRc where Rc represents a hydrogen atom or an alkyl group having 1 to 4 carbon atoms; and A represents any one of
      formulae (1) to (11) 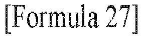 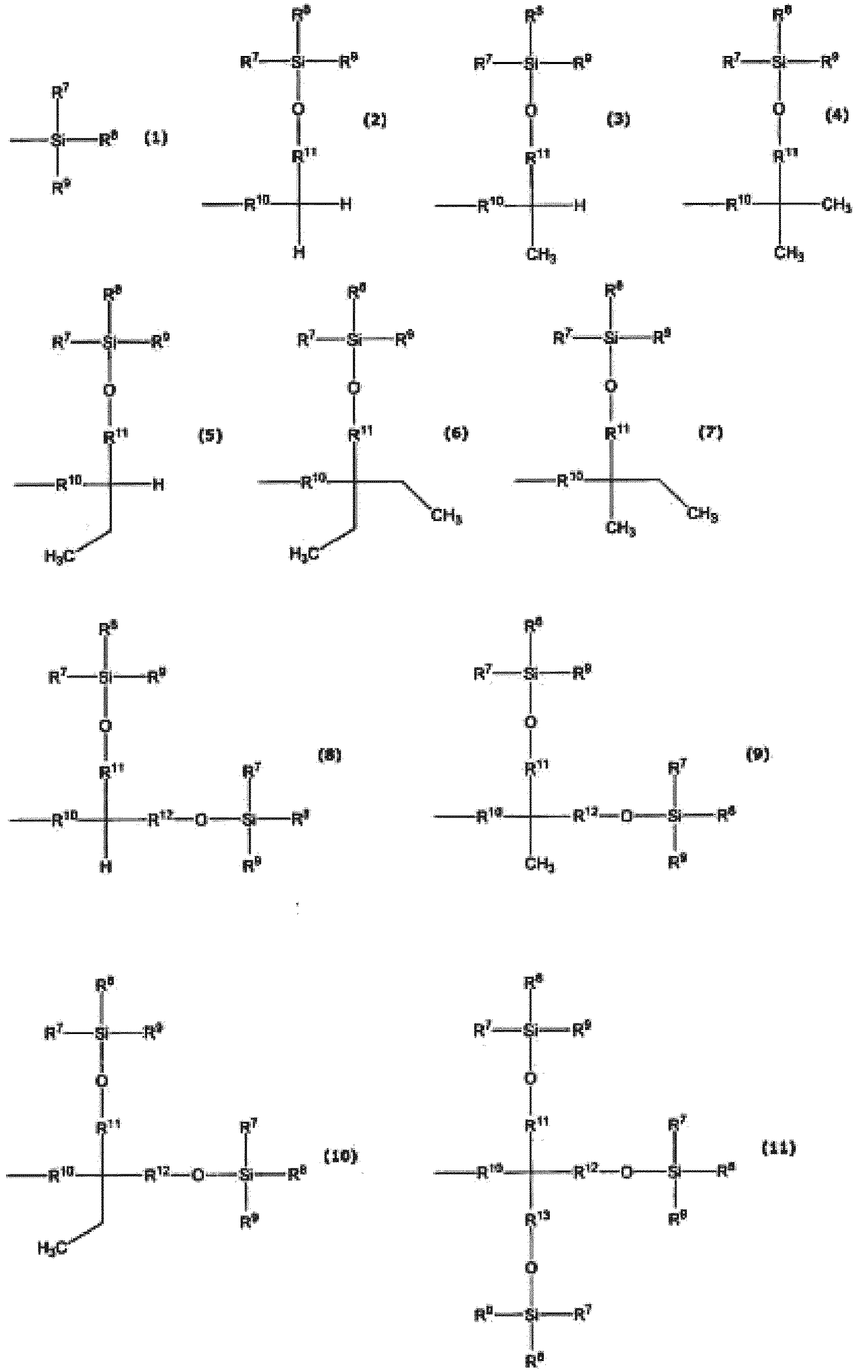
   wherein R⁷, R⁸, and R⁹ are the same as or different from each other and represent a linear or branched alkyl group having 1 to 6 carbon atoms or an aryl group optionally having a substituent, R¹⁰ represents a single bond or a linear or branched alkylene group having 1 to 3 carbon atoms, and R¹¹, R¹², and R¹³ are the same as or different from each other and represent a linear or branched alkylene group having 1 to 3 carbon atoms.

### (Embodiment 4)

The method according to any one of Embodiments 1 to 3, wherein the N-terminal protecting group is selected from the group consisting of a 9-fluorenylmethyloxycarbonyl group (Fmoc group), a *tert*-butoxycarbonyl group (Boc group), a benzyloxycarbonyl group (Cbz group), and an allyloxycarbonyl group (Alloc group).

### (Embodiment 5)

The method according to any one of Embodiments 1 to 4, further comprising, after the condensation step,
a capping step of introducing a reaction mixture containing the N-terminally and C-terminally protected peptide extended N-terminally and a capping agent having an amino group into a flow reactor, thereby reacting an unreacted C-terminally activated derivative of the N-terminally protected amino acid or peptide with the capping agent in the flow reactor to inactivate the C-terminally activated derivative.

### (Embodiment 6)

The method according to Embodiment 5, wherein the capping agent having an amino group is a compound selected from the group consisting of 2-(2-aminoethoxyethanol) (AEE) and 2-aminoethyl hydrogen sulfate (AEHS).

### (Embodiment 7)

An apparatus for producing a peptide, comprising:
a C-terminal activation unit comprising a flow reactor and a raw material introducing member for introducing an N-terminally protected amino acid or peptide, in which an N-terminal amino group is protected by an N-terminal protecting group, and a C-terminal activator into the flow reactor, for activating a C-terminal carboxyl group of the N-terminally protected amino acid or peptide in the flow reactor to form a C-terminally activated derivative of the N-terminally protected amino acid or peptide;
a condensation unit comprising a flow reactor and a raw material introducing member for introducing the C-terminally activated derivative of the N-terminally protected amino acid or peptide and a C-terminally protected amino acid or peptide, in which a C-terminal carboxyl group is protected by a tag protecting group, into the flow reactor, for condensing the N-terminally protected amino acid or peptide with the C-terminally protected amino acid or peptide on the N-terminal side in the flow reactor to form an N-terminally and C-terminally protected peptide extended N-terminally;
an N-terminal deprotection unit comprising a flow reactor and a raw material introducing member for introducing the N-terminally and C-terminally protected peptide extended N-terminally and a deprotecting agent for an N-terminal protecting group into the flow reactor, for removing the N-terminal protecting group for deprotection in the flow reactor to form a C-terminally protected peptide extended N-terminally; and
an extraction unit comprising a mixing member for mixing a reaction mixture containing the C-terminally protected peptide extended N-terminally with an aqueous phase, for mixing the reaction mixture containing the C-terminally protected peptide extended N-terminally with the aqueous phase to extract the C-terminally protected peptide extended N-terminally into an organic phase.

### (Embodiment 8)

The apparatus according to Embodiment 7, further comprising
a capping unit comprising a flow reactor and a raw material introducing member for introducing a reaction mixture containing the N-terminally and C-terminally protected peptide extended N-terminally and a capping agent having an amino group into the flow reactor, for reacting an unreacted C-terminally activated derivative of the N-terminally protected amino acid or peptide with the capping agent in the flow reactor to inactivate the C-terminally activated derivative.

### (Embodiment 9)

The apparatus according to Embodiment 7 or 8, wherein the C-terminal activation unit, the condensation unit, and the N-terminal deprotection unit each comprises two or more inlets, a mixer for mixing a reaction liquid flowing in from the two or more inlets, one or more outlets for allowing the reaction liquid mixed in the mixer to flow out, a pump for feeding a raw material, and a temperature control device for controlling temperatures of the inlets, the outlets, the mixer, and a flow channel of the pump.

### (Embodiment 10)

The apparatus according to any one of Embodiments 7 to 9, wherein the extraction unit comprises two or more inlets, a mixer for mixing a reaction liquid containing the C-terminally protected peptide extended N-terminally flowing in from the two or more inlets with the aqueous phase, a phase separating member for separating a mixed phase of the reaction liquid containing the C-terminally protected peptide extended N-terminally and the aqueous phase, which are mixed in the mixer, into an organic phase and an aqueous phase, one or more outlets for discharging the organic phase separated by the phase separating member, and a pump for feeding the aqueous phase.

### (Embodiment 11)

The apparatus according to Embodiment 9 or 10, wherein at least one of the C-terminal activation unit, the condensation unit, and the N-terminal deprotection unit comprise at least one physical property detecting member selected from the group consisting of a pressure gauge, a flowmeter, and a thermometer in the middle of the inlet and/or the outlet.

### (Embodiment 12)

The apparatus according to Embodiment 10 or 11, wherein the extraction unit comprises at least one physical property detecting member selected from the group consisting of a pressure gauge, a flowmeter, and a thermometer in the middle of the inlet and/or the outlet.

### (Embodiment 13)

The apparatus according to any one of Embodiments 7 to 12, wherein at least one of the C-terminal activation unit, the condensation unit, the N-terminal deprotection unit, and the extraction unit comprises at least one compound detecting member for detecting a compound contained in the raw material and the reaction liquid.

### (Embodiment 14)

The apparatus according to Embodiment 13, wherein the at least one compound detecting member is at least one member selected from the group consisting of an ultraviolet-visible spectroscopy detector, a photodiode array detector, a fluorescence detector, a differential refractive index detector, an electrical conductivity detector, an evaporative light scattering detector, an infrared spectrometer, a near-infrared spectrometer, a nuclear magnetic resonance device, and a mass spectrometer.

### (Embodiment 15)

The apparatus according to Embodiment 13 or 14, further comprising a compound separating member for separating a compound to be detected by the compound detecting member at a front part of the compound detecting member in a flow channel of, for example, the raw material and the reaction liquid.

### (Embodiment 16)

The apparatus according to any one of Embodiments 7 to 15, wherein at least one of the C-terminal activation unit, the condensation unit, the N-terminal deprotection unit, and the extraction unit further comprises at least one outlet port for sampling the reaction liquid.

### (Embodiment 17)

The apparatus according to any one of Embodiments 7 to 16, further comprising a circulating flow unit for circulating and flowing the C-terminally protected peptide extended N-terminally obtained in the extraction unit into the raw material introducing member in the condensation step.

### Advantageous Effect of Invention

According to the present invention, it is possible to provide a unit for producing a peptide with a small number of steps in a short time.

### BRIEF DESCRIPTION OF THE DRAWINGS

FIG. 1 is a flowchart showing each step in one embodiment of a method for producing a peptide according to one aspect of the present invention;
FIG. 2 is a schematic diagram showing one embodiment of an apparatus for producing a peptide according to one aspect of the present invention; and
FIG. 3 is a schematic diagram showing another embodiment of an apparatus for producing a peptide according to one aspect of the present invention.

### DETAILED DESCRIPTION

Hereinafter, preferred embodiments of the present invention are described in detail.

### <1. Method for Producing Peptide>

The present inventors have found that, in a method for producing a peptide using flow reactors, a deprotection reaction of an N-terminal protecting group can be continuously performed without purification of a peptide after a condensation reaction by using a tag protecting group capable of separating and purifying a product by two phase separation of an organic phase and an aqueous phase as a C-terminal protecting group, and a peptide can be thus produced in a short time. Therefore, one aspect of the present invention relates to a method for producing a peptide.

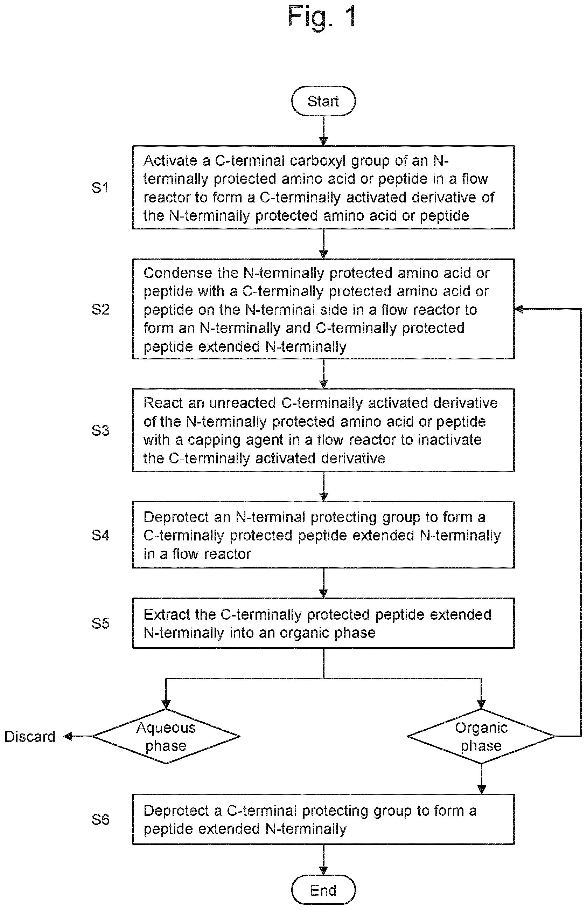

FIG. 1 shows a flowchart showing each step in one embodiment of the method of the present aspect. As shown in FIG. 1, the method of the present aspect includes a C-terminal activation step S1, a condensation step S2, an N-terminal deprotection step S4, and an extraction step S5. The method of the present aspect optionally includes a capping step S3 and a C-terminal deprotection step S6, if desired. Each step is described in detail below.

### [1-1. C-Terminal Activation Step (S1)]

This step includes introducing an N-terminally protected amino acid or peptide, in which an N-terminal amino group is protected by an N-terminal protecting group, and a C-terminal activator into a flow reactor, thereby activating a C-terminal carboxyl group of the N-terminally protected amino acid or peptide in the flow reactor to form a C-terminally activated derivative of the N-terminally protected amino acid or peptide.

Amino acids making up the N-terminally protected amino acid or peptide used in this step may be either natural amino acids or unnatural amino acids. When these amino acids have a side chain functional group, they preferably have a side chain protecting group that protects the functional group. Any amino acids and peptides in which two or more of the amino acids are linked by a peptide bond can be used in this step. The chain length of the N-terminally protected peptide used in this step is usually 2 or more, for example, in the range of 2 to 50, particularly 2 to 30.

The N-terminally protected amino acid or peptide used in this step has an N-terminal amino group, especially an N-terminal α-amino group, protected by an N-terminal protecting group. Examples of the N-terminal protecting group include, but are not limited to, a 9-fluorenylmethyloxycarbonyl group (Fmoc group), a tert-butoxycarbonyl group (Boc group), a benzyloxycarbonyl group (Cbz group), and an allyloxycarbonyl group (Alloc group). The N-terminal protecting group is preferably an Fmoc group or Boc group, more preferably an Fmoc group. The Fmoc group or Boc group can be removed for deprotection by base or acid treatment. In the case of the Fmoc group or Boc group, therefore, the deprotection reaction can be easily carried out by base or acid treatment in a flow reactor in the N-terminal deprotection step described below, as compared to a protecting group (e.g., a Cbz group) in which the deprotection reaction is carried out by catalytic reduction using a catalytic metal powder. The Boc group is sometimes preferably applied to the N-terminal protection of an amino acid or peptide that do not contain cysteine or methionine. However, it is particularly preferable to use the Fmoc group as the N-terminal protecting group because it can be applied to the N-terminal protection of various amino acids or peptides. The peptide can be produced in a small number of steps in a short time by carrying out the method of the aspect using the N-terminal protecting group as illustrated above.

The N-terminally protected amino acid or peptide used in this step may be prepared, for example, by purchasing a commercially available product, or may be prepared by self-synthesis based on a known method.

The C-terminal activator used in this step refers to a compound capable of reacting with the C-terminal carboxyl group of an amino acid or peptide to form a highly electrophilic activated derivative. The C-terminal activator is usually selected from the group consisting of a halogenated alkyl formate, a carboxylic acid halide, phosgene and a phosgene equivalent, and a C-terminally activated condensing agent. The C-terminal activator is preferably selected from the group consisting of halogenated ethyl formate, halogenated isopropyl formate, halogenated isobutyl formate, a carboxylic acid halide, phosgene and a phosgene equivalent, a carbodiimide condensing agent, a phosphonium condensing agent, an uronium condensing agent, a halouronium condensing agent, an imidazole condensing agent, a triazine condensing agent, and a condensation additive, more preferably selected from the group consisting of halogenated alkyl formate, such as halogenated ethyl formate, halogenated isopropyl formate, and halogenated isobutyl formate; a carboxylic acid halide; phosgene and a phosgene equivalent; a carbodiimide condensing agent, such as dicyclohexylcarbodiimide (DCC), diisopropylcarbodiimide (DIC), and N-ethyl-N'-3-dimethylaminopropylcarbodiimide and a hydrochloride thereof (EDC·HCl); a phosphonium condensing agent, such as (benzotriazol-1-yloxy)tripyrrolidinophosphonium hexafluorophosphate (PyBop); an uronium condensing agent, such as [O-(7-azabenzotriazol-1-yl)-1,1,3,3-tetramethyluronium hexafluorophosphate] (HATU), O-(benzotriazol-1-yl)-N,N,N',N'-tetramethyluronium tetrafluoroborate (TBTU), and (1-cyano-2-ethoxy-2-oxoethylidenaminooxy)dimethylamino-morpholino-carbenium hexafluorophosphate (COMU); a halouronium condensing agent, such as 2-chloro-1,3-dimethylimidazolinium hexafluorophosphate (CIP) and 1-(chloro-1-pyrrolidinylmethylene)pyrrolidinium hexafluorophosphate (PyCIU); an imidazole condensing agent, such as N,N'-carbonyldiimidazole (CDI) and 1,1'-carbonyldi(1,2,4-triazole) (CDT); a triazine condensing agent, such as 4-(4,6-dimethoxy-1,3,5-triazin-2-yl)-4-methylmorpholinium chloride (DMT-MM); and a condensation additive, such as 1-hydroxybenzotriazole (HOBt) and 1-hydroxy-7-azabenzotriazol (HOAt), even more preferably selected from the group consisting of isobutyl chloroformate (IBCF), isopropyl chloroformate, ethyl chloroformate, isobutyl bromoformate, isopropyl bromoformate, ethyl bromoformate, triphosgene, diphosgene, thiophosgene, carbonyldiimidazole, pivaloyl chloride, adamantanecarbonyl chloride, isostearoyl chloride, pivaloyl bromide, adamantanecarbonyl bromide, and isostearoyl bromide, and particularly preferably IBCF. The C-terminal activator illustrated above may be used alone or in combination of two or more. The C-terminal activator may be used in combination with a nucleophilic base catalyst such as N,N-dimethylaminopyridine or N-methylimidazole. In the art, condensation of amino acids or peptides usually involves the use of a C-terminally activated condensing agent such as EDC·HCl in combination with a condensation additive such as HOBt. Such a C-terminally activated condensing agent used can react with the C-terminal carboxyl group of an amino acid or peptide to form a relatively mild electrophilic activated derivative. Thus, when the condensation reaction is carried out using a C-terminally activated condensing agent, a peptide bond can be formed in a stereoselective manner while avoiding racemization. When the condensation reaction is carried out using halogenated alkyl formate, a carboxylic acid halide, phosgene, or a phosgene equivalent among C-terminal activators illustrated above, by-products produced after the condensation reaction are, for example, carbon dioxide and hydrogen chloride, and a reaction liquid containing the peptide can be used in the next step as it is without purifying the peptide of the product from the reaction liquid. Furthermore, the C-terminal activator illustrated above is likely to undergo racemization because it can form a highly electrophilic activated derivative. However, in the method of the present aspect, since the condensation reaction is carried out in a flow reactor in which the reaction can proceed in a short time in the condensation step described below, racemization can be substantially avoided. The peptide can be thus produced in a small number of steps in a short time by carrying out the method of the aspect using the C-terminal activator as illustrated above.

The C-terminal activator used in this step may be prepared, for example, by purchasing a commercially available product, or may be prepared by self-synthesis based on a known method.

The C-terminal activator used in this step usually reacts with a carboxyl group under basic conditions. Therefore, the C-terminal activator is preferably reacted with an N-terminally protected amino acid or peptide in the presence of a base. Examples of the base include, but are not limited to, a base having low nucleophilicity such as trialkylamine. The base is preferably N,N-diisopropylethylamine (DIEA), tributylamine, triethylamine, or the like, and more preferably DIEA or tributylamine. The base illustrated above is introduced into a flow reactor together with the N-terminally protected amino acid or peptide or the C-terminal activator, preferably together with the N-terminally protected amino acid or peptide. Alternatively, the base may be introduced into a flow reactor independently of each other without being introduced into the flow reactor together with the N-terminally protected amino acid or peptide or the C-terminal activator, and then mixed using a mixer to prepare a solution inside the flow reactor. The C-terminal activator can be reacted with the N-terminally protected amino acid or peptide in the presence of the base illustrated above, thereby forming an activated derivative with high efficiency.

This step can be performed by introducing a solution containing an N-terminally protected amino acid or peptide in a solvent and a solution containing a C-terminal activator in a solvent into a flow reactor at a predetermined flow velocity. Examples of the solvent include, but are not limited to, a hydrophilic organic solvent, such as N,N-dimethylformamide (DMF), dimethyl sulfoxide (DMSO), tetrahydrofuran (THF), isopropyl acetate, and acetonitrile; and a hydrophobic organic solvent, such as cyclopentyl methyl ether (CPME), 2-methyltetrahydrofuran, 4-methyltetrahydropyran (MTHP), dichloromethane, and t-butyl methyl ether (MTBE). The solution containing an N-terminally protected amino acid or peptide preferably contains the hydrophilic organic solvent illustrated above as a solvent, and more preferably contains DMF as a solvent. The solution containing a C-terminal activator preferably contains the hydrophobic organic solvent illustrated above as a solvent, and more preferably contains CPME or MTHP as a solvent. By using the solvent illustrated above, it is possible to successfully prepare the solution containing an N-terminally protected amino acid or peptide and the solution containing a C-terminal activator.

The base and solvent used in this step may be prepared, for example, by purchasing a commercially available product.

In the case of the present embodiment, the solution containing an N-terminally protected amino acid or peptide preferably contains a base as illustrated above. In the solution containing an N-terminally protected amino acid or peptide, the amount of the N-terminally protected amino acid or peptide is preferably in the range of 1.0 to 5.0 molar equivalents, and more preferably in the range of 1.0 to 1.3 molar equivalents, with respect to the C-terminally protected amino acid or peptide. The amount of the base is preferably in the range of 1.0 to 5.0 molar equivalents, and more preferably in the range of 1.0 to 1.3 molar equivalents, with respect to the C-terminally protected amino acid or peptide. In the solutions containing a C-terminal activator, the amount of the C-terminal activator is preferably in the range of 1.0 to 5.0 molar equivalents, and more preferably in the range of 1.0 to 1.3 molar equivalents, with respect to the C-terminally protected amino acid or peptide. When the amount of the components is less than the lower limit, the formation rate of the activated derivative may decrease. When the amount of the components exceeds the upper limit, these components may not be sufficiently dissolved in the solvent, resulting in deposition in a flow reactor. The deposition of the components may cause clogging of a flow reactor. By carrying out this step in an amount in the above range, it is possible to form an activated derivative with high efficiency and produce a peptide in a short time without clogging of a flow reactor.

In the case of the embodiment, the flow velocity at which the solution containing an N-terminally protected amino acid or peptide is introduced into a flow reactor is preferably in the range of 0.1 to 100 mL/min, and more preferably in the range of 0.5 to 20 mL/min. The flow velocity at which the solution containing a C-terminal activator is introduced into a flow reactor is preferably in the range of 0.1 to 100 mL/min, and more preferably in the range of 0.5 to 20 mL/min. When the flow velocity is less than the lower limit, the time required to produce a peptide by the method of the present aspect may be longer. By carrying out this step at a flow velocity in the above range, it is possible to form an activated derivative with high efficiency and produce a peptide in a short time.

In this step, the temperature of the formation reaction of the activated derivative in a flow reactor is preferably in the range of 0 to 100°C, and more preferably in the range of 20 to 60°C. When the temperature of the activation reaction is less than the lower limit, the yield of the formation reaction of the activated derivative may decrease. In addition, when the temperature of the formation reaction of the activated derivative exceeds the upper limit, a by-product may be formed. By carrying out this step at a temperature in the above range, it is possible to form a C-terminally activated derivative of the N-terminally protected amino acid or peptide in high yield.

In this step, the time of the formation reaction of the activated derivative in a flow reactor is preferably in the range of 0.05 seconds to 30 minutes, and more preferably in the range of 0.1 to 30 seconds. When the time of the formation reaction of the activated derivative is less than the lower limit, the yield of the formation reaction of the activated derivative may decrease. In addition, when the time of the formation reaction of the activated derivative exceeds the upper limit, a by-product may be formed. By carrying out this step within the above time range, it is possible to form a C-terminally activated derivative of the N-terminally protected amino acid or peptide in high yield.

By carrying out this step under the above conditions, it is possible to activate the C-terminal carboxyl group of the N-terminally protected amino acid or peptide in a flow reactor to form a C-terminally activated derivative of the N-terminally protected amino acid or peptide.

### [1-2. Condensation Step (S2)]

This step includes introducing a C-terminally activated derivative of the N-terminally protected amino acid or peptide and a C-terminally protected amino acid or peptide in which a C-terminal carboxyl group is protected by a tag protecting group, into a flow reactor, thereby condensing the N-terminally protected amino acid or peptide with the C-terminally protected amino acid or peptide on the N-terminal side in the flow reactor to form an N-terminally and C-terminally protected peptide extended N-terminally.

The C-terminally activated derivative of the N-terminally protected amino acid or peptide used in this step is obtained in the C-terminal activation step described above.

Amino acids making up the C-terminally protected amino acid or peptide used in this step may be either natural amino acids or unnatural amino acids. When these amino acids have a side chain functional group, they preferably have a side chain protecting group that protects the functional group. Any amino acids and peptides in which two or more of the amino acids are linked by a peptide bond can be used in this step. The chain length of the N-terminally protected peptide used in this step is usually 2 or more, for example, in the range of 2 to 50, particularly 2 to 30.

The C-terminally protected amino acid or peptide used in this step has a C-terminal carboxyl group protected by a tag protecting group. The tag protecting group used in this step refers to a protecting group on the C-terminal carboxyl group of an amino acid or peptide, which reduces its polarity so that the amino acid or peptide or a condensation product thereof can be separated and purified by two phase separation in organic and aqueous phases. Examples of the tag protecting group include, but are not limited to, a protecting group composed of a benzyl compound described in International Publication No. WO 2017/038650 and a pseudo-solid-phase protecting group described in International Publication No. WO 2020/218497.

The tag protecting group is preferably a monovalent group derived from a compound selected from the group consisting of compounds represented by formulae (I) to (IX) below:
(i) a fluorene compound represented by formula (I): 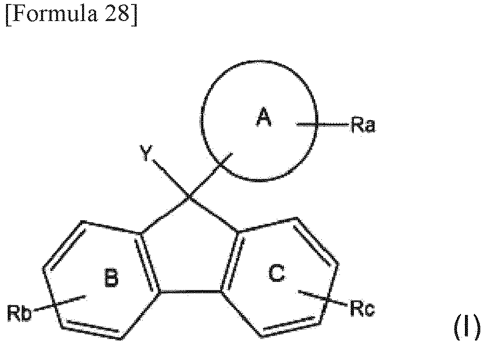 wherein
   Ring A represents an aromatic ring; Y is a hydroxyl group, a bromo group, or a chloro group; Ra, Rb, and Rc each independently represent an organic group having an aliphatic hydrocarbon group, a hydrogen atom, or an electron withdrawing group, and at least one of Ra, Rb, and Rc is an organic group having an aliphatic hydrocarbon group; and Rings A, B and C each independently optionally have an electron withdrawing group,
   wherein the organic group having an aliphatic hydrocarbon group is at least one group selected from the group consisting of
   a group represented by formula (b): 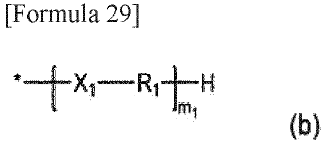 wherein
      * represents a binding position; X₁ is -O-; R₁ is an aliphatic hydrocarbon group having 5 to 60 carbon atoms; and m₁ is 1,
      a group represented by formula (c): 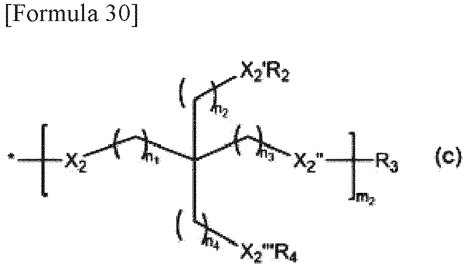 wherein
         * represents a binding position; X₂, X₂', X₂", and X₂‴ are -O-; R₂ and R₄ are each independently an aliphatic hydrocarbon group having 5 to 60 carbon atoms; R₃ is an organic group having an aliphatic hydrocarbon group having 5 to 60 carbon atoms; n₁, n₂, n₃, and n₄ are 1; and m₂ is 1, and
         a group represented by formula (d): 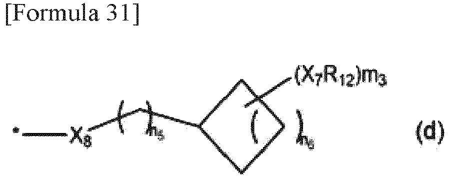 wherein
            * represents a binding position; X₈ represents -O-; m₃ is 2 or 3; n₅ is 1; n₆ is 3; X₇ is - O-; m₃ number of R₁₂ are each independently an alkyl group having 4 to 30 carbon atoms,
            wherein the organic group having an aliphatic hydrocarbon group is present at a 2- and/or 7-position of the fluorene compound,
(ii) a fluorene compound represented by formula (II): 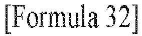 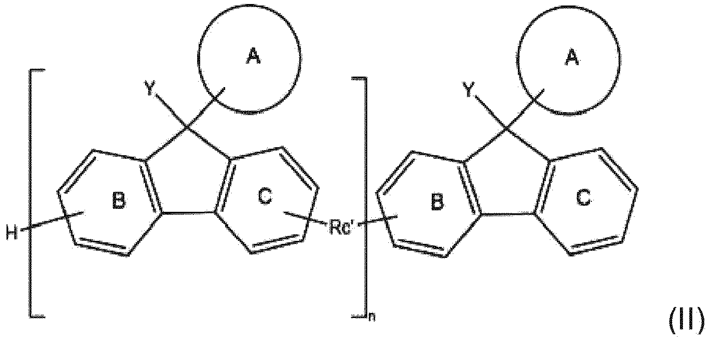 wherein
   Ring A represents an aromatic ring; Y is a hydroxyl group, a bromo group, or a chloro group; n represents an integer of 1 to 19; Rc' is a divalent organic group having an aliphatic hydrocarbon group; Rings A, B, and C each independently optionally have at least one selected from an organic group having an aliphatic hydrocarbon group and an electron withdrawing group; when a plurality of Rings A are present, Rings A are the same as or different from each other; when a plurality of Y are present, Y are the same as or different from each other; and when a plurality of Rc' are present, Rc's are the same as or different from each other,
   wherein the divalent organic group having an aliphatic hydrocarbon group is a group represented by a group represented by formula (a): 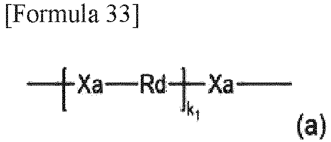 wherein
      Xa is absent or represents -O-, -S-, -NHCO-, or -CONH-; Rd represents an aliphatic hydrocarbon group having 5 or more carbon atoms; k1 represents an integer of 1 to 10; when a plurality of Rd are present, Rd are the same as or different from each other; and when a plurality of Xa are present, Xa are the same as or different from each other, and
      wherein the organic group having an aliphatic hydrocarbon group is at least one group selected from the group consisting of
      a group represented by formula (b): 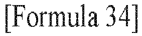 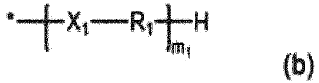 wherein
         * represents a binding position; X₁ is -O-; R₁ is an aliphatic hydrocarbon group having 5 to 60 carbon atoms; and m₁ is 1,
         a group represented by formula (c): 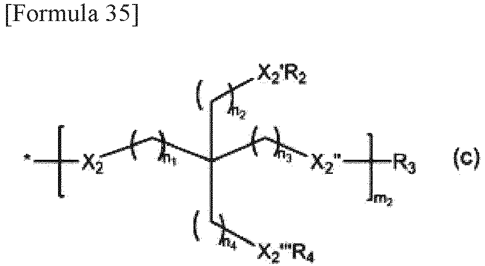 wherein
            * represents a binding position; X₂, X₂', X₂", and X₂‴ are -O-; R₂ and R₄ are each independently an aliphatic hydrocarbon group having 5 to 60 carbon atoms; R₃ is an organic group having an aliphatic hydrocarbon group having 5 to 60 carbon atoms; n₁, n₂, n₃, and n₄ are 1; and m₂ is 1, and
            a group represented by formula (d): 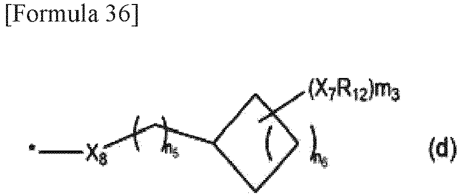 wherein
               * represents a binding position; X₈ represents -O-; m₃ is 2 or 3; n₅ is 1; n₆ is 3; X₇ is - O-; m₃ number of R₁₂ are each independently an alkyl group having 4 to 30 carbon atoms,
               wherein the organic group having the aliphatic hydrocarbon group is present at a 2- and/or 7-position of the fluorene compound,
(iii) a benzyl compound represented by formula (III): 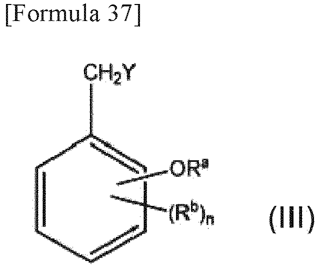 wherein
   Y represents a hydroxyl group or an -NHR group, where R represents a hydrogen atom, an alkyl group, or an aralkyl group;
   R^{a} is an organic group having an aliphatic hydrocarbon group selected from the group consisting of
   a group represented by formula (a): 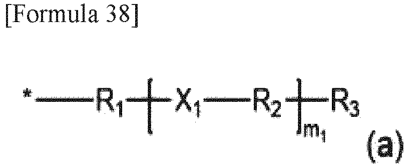 wherein
      * represents a binding position; m₁ represents an integer of 1 to 10; m₁ number of X₁ each independently represent a single bond or -O-, -S-, -COO-, -OCONH-, -NHCO-, or - CONH-; R₁ and m₁ number of R₂ each independently represent a divalent aliphatic hydrocarbon group having 5 or more carbon atoms; and R₃ is a hydrogen atom, or
      a group represented by formula (III'): 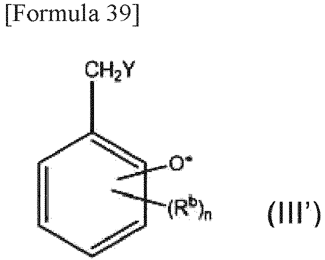 wherein
         Y represents a hydroxyl group or an -NHR group, where R represents a hydrogen atom, an alkyl group, or an aralkyl group; * represents a binding position; n number of R^{b} each independently represent an alkoxy group having 1 to 6 carbon atoms, a halogen atom, or an alkyl group having 1 to 6 carbon atoms optionally substituted with one or more halogen atoms; and n represents an integer of 0 to 4;
         a group represented by formula (b): 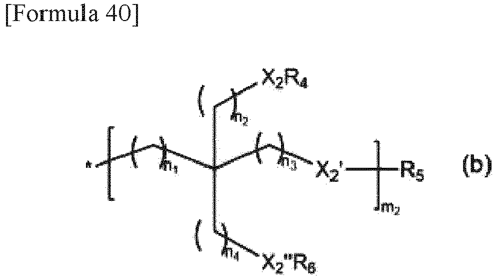 wherein
            * represents a binding position; m2 represents 1 or 2; n₁, n₂, n₃, and n₄ each independently represent an integer of 0 to 2; m₂ number of X₂, m₂ number of X₂', and m₂ number of X₂" each independently represent a single bond or -O-, -S-, -COO-, -OCONH-, - NHCO-, or -CONH-; m₂ number of R₄ and m₂ number of R₆ each independently represent an aliphatic hydrocarbon group having 5 or more carbon atoms; and R₅ represents an aliphatic hydrocarbon group having 5 or more carbon atoms;
            a group represented by formula (c): 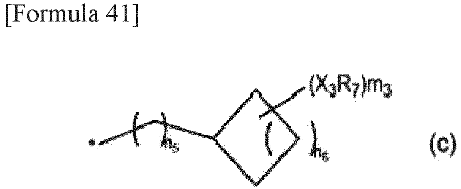 wherein
               * represents a binding position; m₃ represents an integer of 0 to 15; n₅ represents an integer of 0 to 11; n₆ represents an integer of 0 to 5; m₃ number of X₃ each independently represent a single bond or -O-, -S-, -COO-, -OCONH-, -NHCO-, or -CONH-; and m₃ number of R₇ each independently represent a hydrogen atom, a methyl group, or an aliphatic hydrocarbon group having 5 or more carbon atoms; and
               a group represented by formula (d): 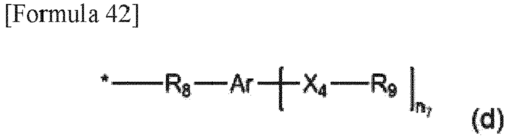 wherein
                  * represents a binding position; n₇ number of X₄ each independently represent a single bond or -O-, -S-, -COO-, -OCONH-, -NHCO-, or -CONH-; R₈ represents a divalent aliphatic hydrocarbon group; n₇ number of R₉ each independently represent a monovalent aliphatic hydrocarbon group; n₇ represents an integer of 1 to 5; and Ar represents an arylene group,
                  the organic group having a total number of carbon atoms of 30 or more;
                  n number of _{R}b each independently represent an alkoxy group having 1 to 6 carbon atoms, a halogen atom, or an alkyl group having 1 to 6 carbon atoms optionally substituted with one or more halogen atoms; and n represents an integer of 0 to 4,
(iv) a compound represented by formula (IV): 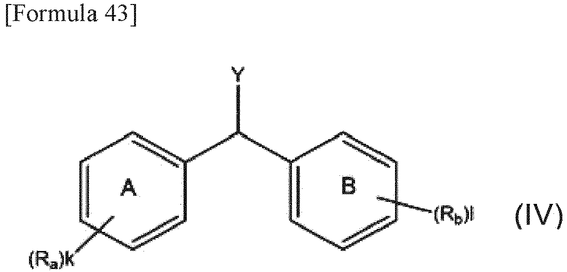 wherein
   Y represents a hydroxyl group or an -NHR group, where R represents a hydrogen atom, an alkyl group, or an aralkyl group; k and l each independently represent an integer of 0 to 5, provided that k+1 is not 0; k number of Rₐ and one R_{b} each independently represent an organic group having an aliphatic hydrocarbon group having 5 or more carbon atoms selected from the group consisting of
   a group represented by formula (a): 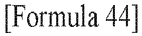 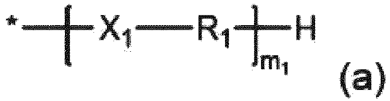 wherein
      * represents a binding position; m₁ represents an integer of 1 to 10; m₁ number of X₁ each independently are absent or represent -O-, -S-, -COO-, -OCONH-, or -CONH-; m₁ number of R₁ each independently represent a divalent aliphatic hydrocarbon group having 5 or more carbon atoms,
      a group represented by formula (b): 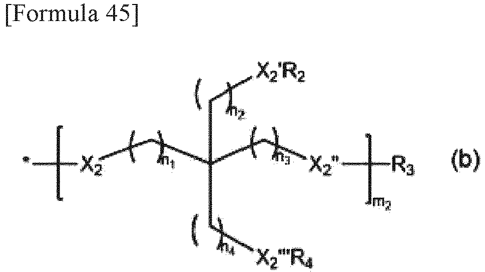 wherein
         * represents a binding position; m₂ represents an integer of 1 or 2; m₂ number of n₁, n₂, n₃, and n₄ each independently represent an integer of 0 to 2; m₂ number of X₂, m₂ number of X₂', m₂ number of X₂‴, and m₂ number of X₂" each independently are absent or represent -O-, -S-, -COO-, -OCONH-, or -CONH-; m₂ number of R₂ and R₄ each independently represent a hydrogen atom, a methyl group, or an aliphatic hydrocarbon group having 5 or more carbon atoms; and R₃ represents an aliphatic hydrocarbon group having 5 or more carbon atoms, and
         a group represented by formula (e): 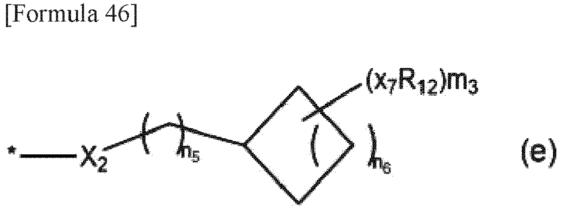 wherein
            * represents a binding position; m₃ represents an integer of 0 to 15; n₅ represents an integer of 0 to 11; n₆ represents an integer of 0 to 5; X₂ is absent or represents -O-, -S-, -NHCO-, or -CONH-; m₃ number of X₇^{s} each independently are absent or represent -O-, -S-, -COO-, - OCONH-, -NHCO-, or -CONH-; and m₃ number of R₁₂ each independently represent a hydrogen atom, a methyl group, or an aliphatic hydrocarbon group having 5 or more carbon atoms,
            wherein the total number of carbon atoms of all aliphatic hydrocarbon groups in an organic group having k+1 number of aliphatic hydrocarbon groups is 16 or more; Ring A optionally further has a substituent in addition to Rₐ; and Ring B optionally further has a substituent in addition to R_{b},
(v) a branched chain-containing aromatic compound represented by formula (V): 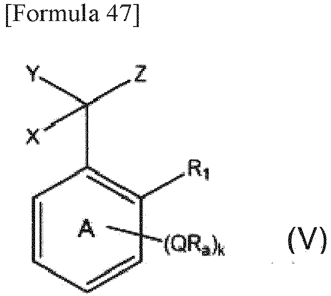 wherein
   k number of Q each independently represent a single bond or -O-, -S-, -C(=O)O-, - C(=O)NH-, or -NH-; k number of Rₐ each independently represent an organic group having at least one aliphatic hydrocarbon group having one or more branched chains with a total number of branched chains of 3 or more and a total number of carbon atoms of 14 or more and 300 or less; k represents an integer of 1 to 4; R₁ is a hydrogen atom or, when Z is a group represented by formula (a) below, represents a single bond together with R₂ to form a fluorene ring together with Ring B; Ring A optionally has at least one substituent selected from the group consisting of a halogen atom, a C1-6 alkyl group optionally substituted with one or more halogen atoms, and a C1-6 alkoxy group optionally substituted with one or more halogen atoms, in addition to R₁, k number of QRa, and C(X)(Y)Z; X represents a hydrogen atom or a phenyl group; Y represents a hydroxyl group or an -NHR group, where R represents a hydrogen atom, an alkyl group or an aralkyl group; and Z represents a hydrogen atom or a group represented by formula (a): 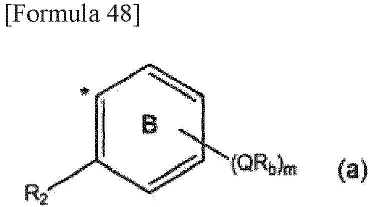 wherein
   * represents a binding position; m represents an integer of 0 to 4; m number of Q each independently represent a single bond or -O-, -S-, -C(=O)O-, -C(=O)NH-, or -NH-; m number of R_{b} each independently represent an organic group having at least one aliphatic hydrocarbon group having one or more branched chains with a total number of branched chains of 3 or more and a total number of carbon atoms of 14 or more and 300 or less; R₂ represents a hydrogen atom or represents a single bond together with R₁ to form a fluorene ring together with Ring A; and Ring B optionally has one or more substituents selected from the group consisting of a halogen atom, a C1-6 alkyl group optionally substituted with one or more halogen atoms, and a C1-6 alkoxy group optionally substituted with one or more halogen atoms, in addition to m number of QR_{b} and R₂; and
   the organic group having at least one aliphatic hydrocarbon group having one or more branched chains with a total number of branched chains of 3 or more and a total number of carbon atoms of 14 or more and 300 or less in Rₐ and R_{b} is a group having 3 or more same or different divalent groups represented by a group represented by formula (b): 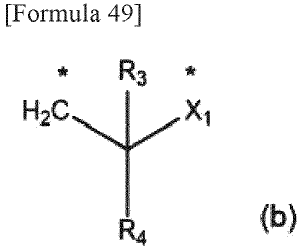 wherein
      * represents a binding position to an adjacent atom; R₃ and R₄ each independently represent a hydrogen atom or a C1-4 alkyl group; and X₁ represents a single bond, a C1-4 alkylene group, or an oxygen atom, provided that R₃ and R₄ are not simultaneously hydrogen atoms,
(vi) a compound represented by formula (VI): 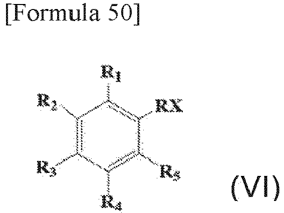 wherein
   R₁ and R₅ are each a hydrogen atom; R₂, R₃, and R₄ are each an alkoxy group having 8 to 30 carbon atoms; RX is a group binding to a carboxyl group, an amino group, or a hydroxy group of an amino acid, a peptide, or an amino acid amide or to a linker, which is represented by -CHO, -CH₂OH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group,
(vii) a compound represented by formula (VII): 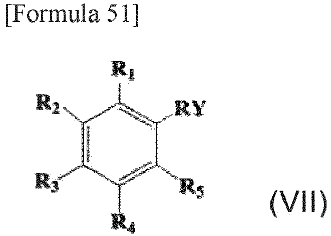 wherein
   R₂, R₄, and R₅ are each a hydrogen atom; R₁ and R₃ are each an alkoxy group having 12 to 30 carbon atoms; RY is a group binding to a carboxyl group, an amino group, or a hydroxy group of an amino acid, a peptide, or an amino acid amide or to a linker, which is represented by -CHO, -CH₂OH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group,
(viii) a compound represented by formula (VIII): 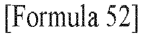 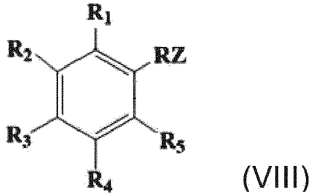 wherein
   R₁, R₃, and R₅ are each a hydrogen atom; R₂ and R₄ are each an alkoxy group having 12 to 30 carbon atoms; RZ is a group binding to a carboxyl group, an amino group, or a hydroxy group of an amino acid, a peptide, or an amino acid amide or to a linker, which is represented by -CHO, -CH₂OH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group, and
(ix) a compound represented by formula (IX): 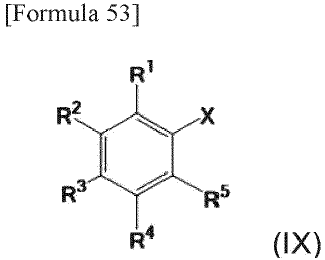 wherein
   X is a group representing -CH₂ORa where Ra represents a hydrogen atom, a halogenocarbonyl group, or an active ester protecting group, -CH₂NHRb where Rb represents a hydrogen atom, a linear or branched alkyl group having 1 to 6 carbon atoms, or an aralkyl group, a halogenomethyl group, a methyl azide group, a formyl group, or an oxime, and binding to a carboxyl group, an amino group, or a hydroxy group of an amino acid, a peptide, or an amino acid amide or to a linker; at least one of R¹, R², R³, R⁴ and R⁵ represents a group represented by a formula of -O-R⁶-Xa-A, and a residual group represents a hydrogen atom, a halogen atom, an alkyl group having 1 to 4 carbon atoms, or an alkoxy group having 1 to 4 carbon atoms; R⁶ represents a linear or branched alkylene group having 1 to 16 carbon atoms, and Xa represents O or CONRc where Rc represents a hydrogen atom or an alkyl group having 1 to 4 carbon atoms; and A represents any one of formulae (1) to (11): 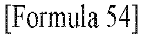 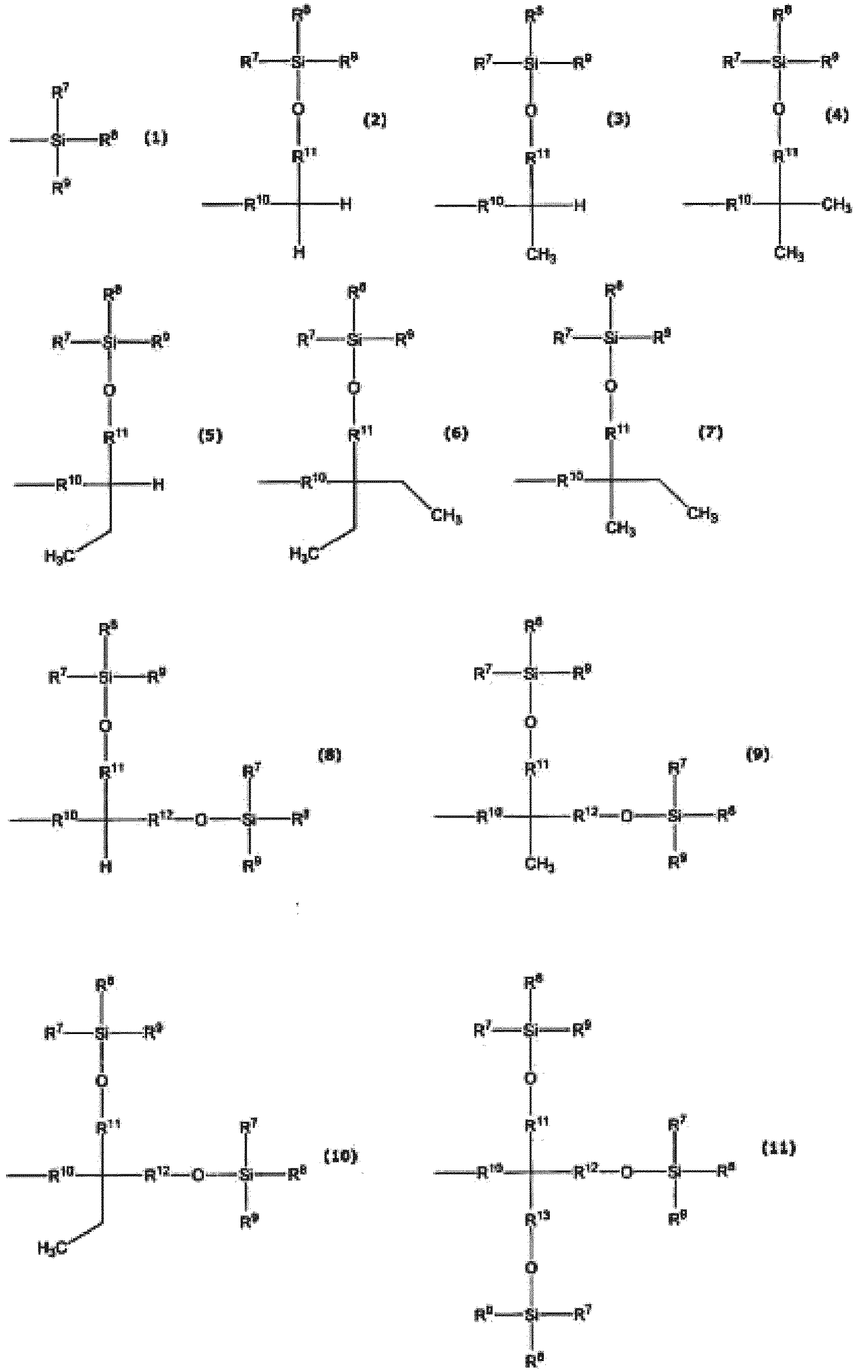 wherein R⁷, R⁸, and R⁹ are the same as or different from each other and represent a linear or branched alkyl group having 1 to 6 carbon atoms or an aryl group optionally having a substituent, R¹⁰ represents a single bond or a linear or branched alkylene group having 1 to 3 carbon atoms, and R¹¹, R¹², and R¹³ are the same as or different from each other and represent a linear or branched alkylene group having 1 to 3 carbon atoms.

The tag protecting is preferably a monovalent group derived from a compound selected from the group consisting of compound represented by formulae TIPS2-OP, TIPS3-OMP, TIPS3-MMP, TIPS3-O, TIPS6-OP, TIPS6-MMP, TIPS9-OMP, and TBDPS2-OP: 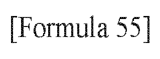 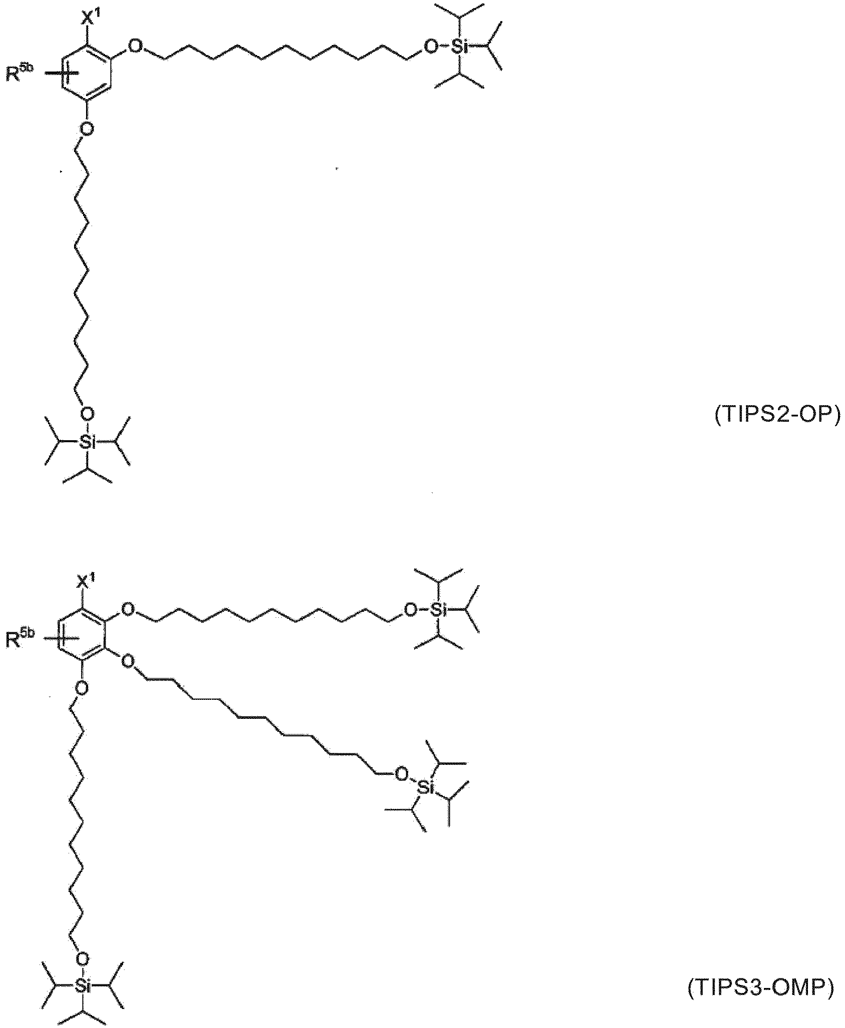 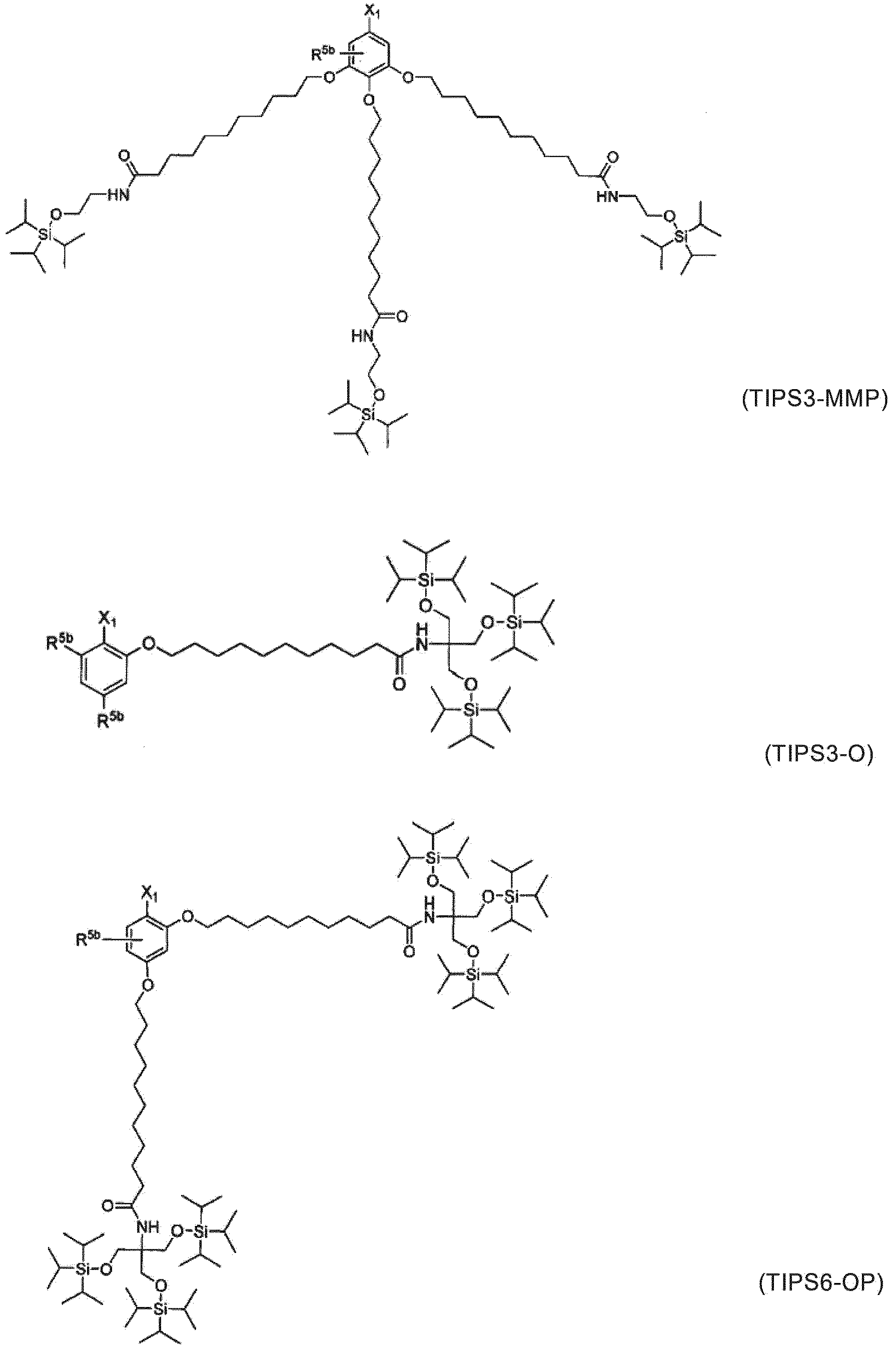 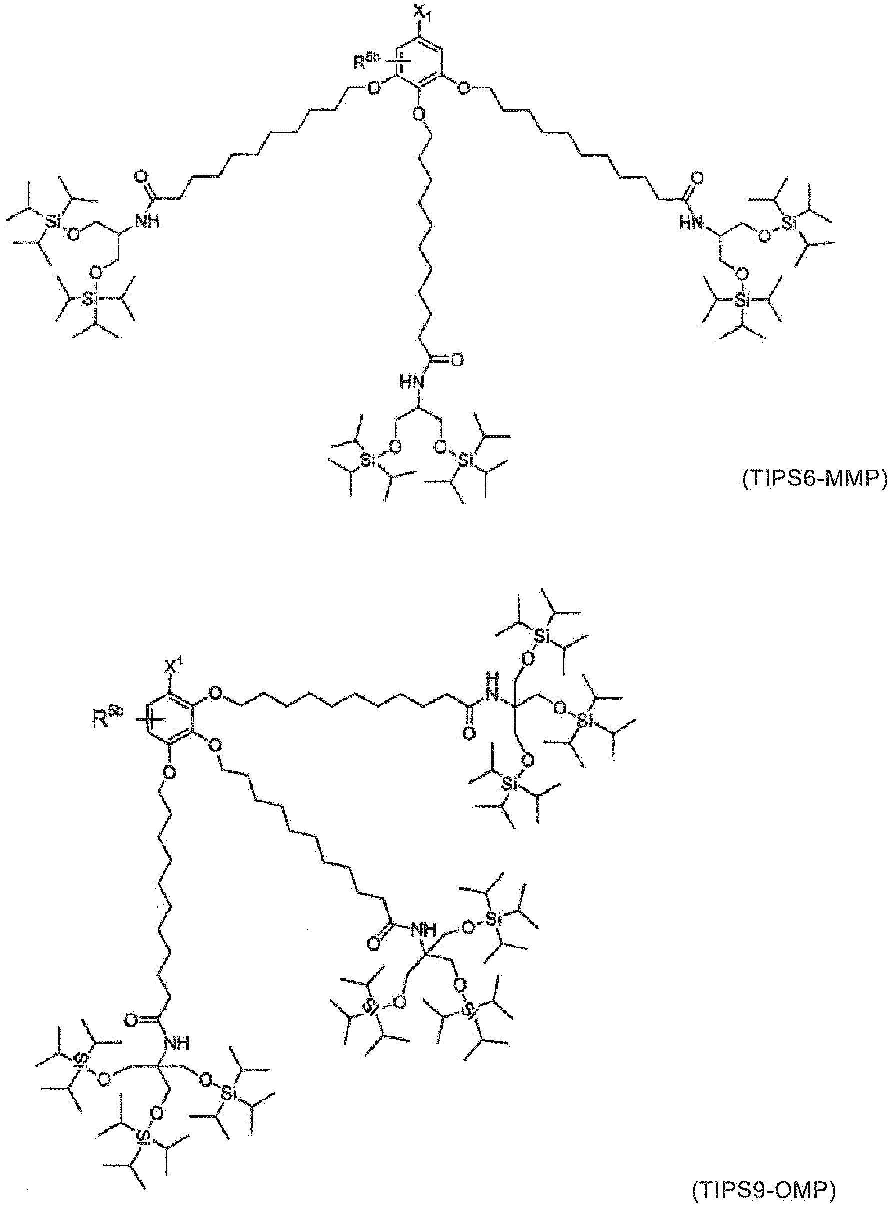 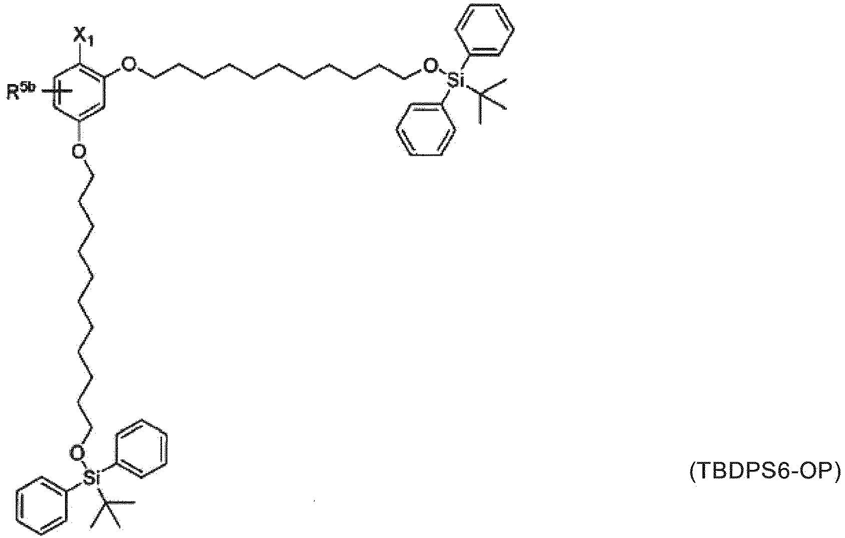 wherein
X¹ is -CH₂OR¹ or -CH₂NHR²;
R¹ is a hydrogen atom, a halogenocarbonyl group, or an active ester protecting group;
R² is a hydrogen atom, a linear or branched alkyl group having 1 to 6 carbon atoms, or an aralkyl group; and
R^{5b} is a hydrogen atom, a halogen atom, an alkyl group having 1 to 4 carbon atoms, or an alkoxy group having 1 to 4 carbon atoms.
The tag protecting group is more preferably a monovalent group derived from TIPS2-OP. In the formulae illustrated above, X¹ is preferably -CH₂OH or -CH₂NH₂, and more preferably -CH₂OH. R^{5b} is preferably a hydrogen atom. Particularly preferred is a compound (TIPS2-OH) in which X¹ is -CH₂OH; and R^{5b} is a hydrogen atom in a formula of TIPS2-OP. The compound illustrated above is described in International Publication No. WO 2017/038650 or International Publication No. WO 2020/218497.

The monovalent group illustrated above is usually a radical obtained by eliminating a hydrogen atom from a hydroxyl group of the corresponding compound. By carrying out this step using the tag protecting group illustrated above, it is possible to substantially avoid deposition of the N-terminally and C-terminally protected peptide extended N-terminally obtained by the condensation reaction in a flow reactor. In the extraction step described below, the C-terminally protected peptide extended N-terminally can be easily extracted by two phase separation of organic and aqueous phases. Therefore, the peptide can be produced in a small number of steps in a short time by the method of the present aspect.

The C-terminally protected amino acid or peptide used in this step may be prepared, for example, by purchasing a commercially available product, or may be prepared by self-synthesis based on known methods described in International Publication No. WO 2017/038650 and International Publication No. WO 2020/218497.

This step can be performed by introducing a solution containing C-terminally activated derivative of the N-terminally protected amino acid or peptide in a solvent and a solution containing a C-terminally protected amino acid or peptide into a flow reactor at a predetermined flow velocity. The solvent can be the same as those illustrated in the description of the C-terminal activation step. The solutions containing a C-terminally activated derivative of the N-terminally protected amino acid or peptide usually contain the solvent used in the C-terminal activation step. The solution containing a C-terminally protected amino acid or peptide preferably contains the hydrophilic organic solvent illustrated above as a solvent, and more preferably contains DMF as a solvent. By using the solvent illustrated above, it is possible to successfully prepare the solution containing a C-terminally activated derivative of the N-terminally protected amino acid or peptide and the solution containing a C-terminally protected amino acid or peptide.

The solvent used in this step may be prepared, for example, by purchasing a commercially available product.

In the case of the present embodiment, as the solution containing a C-terminally activated derivative of the N-terminally protected amino acid or peptide, it is preferable to use the reaction mixture containing a C-terminally activated derivative of the N-terminally protected amino acid or peptide obtained in the C-terminal activation step as it is. The peptide can be produced in a small number of steps in a short time by using the reaction mixture containing a C-terminally activated derivative of the N-terminally protected amino acid or peptide obtained in the C-terminal activation step as it is in this step.

In the case of the present embodiment, in the solution containing a C-terminally activated derivative of the N-terminally protected amino acid or peptide, the amount of the C-terminally activated derivative of the N-terminally protected amino acid or peptide is preferably in the range of 1.0 to 5.0 molar equivalents, and more preferably in the range of 1.0 to 1.3 molar equivalents, with respect to the C-terminally protected amino acid or peptide. In the case of using the reaction mixture containing a C-terminally activated derivative of the N-terminally protected amino acid or peptide obtained in the C-terminal activation step as it is, the amount of the C-terminally activated derivative of the N-terminally protected amino acid or peptide can be varied in the above range depending on the yield of the reaction in the C-terminal activation step. In the solution containing a C-terminally protected amino acid or peptide, the concentration of the C-terminally protected amino acid or peptide is preferably in the range of 0.01 to 1.0 mol/L, and more preferably in the range of 0.05 to 0.3 mol/L. When the amount or concentration of the components is less than the lower limit, the yield of the condensation reaction may decrease. When the amount or concentration of the components exceeds the upper limit, these components may not be sufficiently dissolved in the solvent, resulting in deposition in a flow reactor. The deposition of the components may cause clogging of a flow reactor. By carrying out this step in an amount or concentration in the above range, it is possible to form an activated derivative with high efficiency and produce a peptide in a short time without clogging of a flow reactor.

In the case of the embodiment, the flow velocity at which the solution containing a C-terminally activated derivative of the N-terminally protected amino acid or peptide is introduced into a flow reactor is preferably in the range of 0.1 to 100 mL/min, and more preferably in the range of 0.5 to 20 mL/min. In the case of using the reaction mixture containing a C-terminally activated derivative of the N-terminally protected amino acid or peptide obtained in the C-terminal activation step as it is, the flow velocity of the solution containing a C-terminally activated derivative of the N-terminally protected amino acid or peptide can be varied depending on the flow velocity of each solution in the C-terminal activation step. The flow velocity at which the solution containing a C-terminally protected amino acid or peptide is introduced into a flow reactor is preferably in the range of 0.2 to 200 mL/min, and more preferably in the range of 1 to 40 mL/min. When the flow velocity is less than the lower limit, the time required to produce a peptide by the method of the present aspect may be longer. In addition, when the flow velocity exceeds the upper limit, the yield of the condensation reaction may decrease. By carrying out this step at a flow velocity in the above range, it is possible to form N-terminally and C-terminally protected peptides extended N-terminally in high yield and produce a peptide in a short time.

In this step, the temperature of the condensation reaction between the C-terminally protected amino acid or peptide and the N-terminally protected amino acid or peptide in a flow reactor is preferably in the range of 0 to 100°C, and more preferably in the range of 20 to 60°C. When the temperature of the condensation reaction is less than the lower limit, the yield of the condensation reaction may decrease. In addition, when the temperature of the condensation reaction exceeds the above upper limit, racemization may proceed. A peptide having a desired absolute configuration can be produced in high yield by carrying out this step at a temperature in the above range.

In this step, the time of the condensation reaction between the C-terminally protected amino acid or peptide and the N-terminally protected amino acid or peptide in a flow reactor is preferably in the range of 1 second to 60 minutes, and more preferably in the range of 10 seconds to 5 minutes. When the time of the condensation reaction is less than the lower limit, the yield of the condensation reaction may decrease. In addition, when the time of the condensation reaction exceeds the above upper limit, racemization may proceed. A peptide having a desired absolute configuration can be produced in high yield by carrying out this step within the above time range.

By carrying out this step under the above conditions, it is possible to condense the N-terminally protected amino acid or peptide on an N-terminal side of the C-terminally protected amino acid or peptide in a flow reactor to form an N-terminally and C-terminally protected peptide extended N-terminally.

### [1-3. Capping Step (S3)]

This step includes introducing a reaction mixture containing an N-terminally and C-terminally protected peptide extended N-terminally and a capping agent having an amino group into a flow reactor, thereby reacting an unreacted C-terminally activated derivative of the N-terminally protected amino acid or peptide with the capping agent in the flow reactor to inactivate the C-terminally activated derivative.

This step is preferably performed after the condensation step, and more preferably performed after the condensation step using the reaction mixture containing the N-terminally and C-terminally protected peptide extended N-terminally obtained in the condensation step.

The capping agent used in this step refers to a compound having an amino group, which can react with an unreacted C-terminally activated derivative of the N-terminally protected amino acid or peptide to inactivate the unreacted C-terminally activated derivative of the N-terminally protected amino acid or peptide. The capping agent preferably has an additional hydrophilic group, such as a hydroxyl group or a sulfate group, in addition to the amino group. Having an additional hydrophilic group can improve the water solubility of the reaction product with the C-terminally activated derivative of the N-terminally protected amino acid or peptide. As a result, the inactivated reaction product with the capping agent can be transferred to an aqueous phase in the extraction step described below. The capping agent is preferably selected from the group consisting of 2-(2-aminoethoxyethanol) (AEE) and 2-aminoethyl hydrogen sulfate (AEHS), and more preferably AEE. The unreacted C-terminally activated derivative of the N-terminally protected amino acid or peptide can be rapidly inactivated by using the capping agent illustrated above, whereby the peptide can be produced in a small number of steps in a short time.

The capping agent used in this step may be prepared, for example, by purchasing a commercially available product.

This step can be performed by introducing a reaction mixture containing an N-terminally and C-terminally protected peptide extended N-terminally and a solution containing a capping agent in a solvent into a flow reactor at a predetermined flow velocity. The solvent can be the same as those illustrated in the description of the C-terminal activation step. The reaction mixture containing the N-terminally and C-terminally protected peptide extended N-terminally usually contains the solvent used in the condensation step. The solution containing a capping agent preferably contains the hydrophilic organic solvent illustrated above as a solvent, and more preferably contains DMSO as a solvent. By using the solvent illustrated above, it is possible to successfully prepare the solution containing a capping agent.

The solvent used in this step may be prepared, for example, by purchasing a commercially available product.

In the case of the present embodiment, in a reaction mixture containing an N-terminally and C-terminally protected peptide extended N-terminally, the concentration of the N-terminally and C-terminally protected peptide extended N-terminally is preferably in the range of 0.01 to 1.0 mol/L, and more preferably in the range of 0.05 to 0.3 mol/L. The concentration of the N-terminally and C-terminally protected peptide extended N-terminally can be varied in the above range depending on the yield of the reaction in the condensation step. In the solutions containing a capping agent, the amount of the capping agent is preferably in the range of 0.5 to 20 molar equivalents, and more preferably in the range of 0.5 to 2.0 molar equivalents, with respect to the C-terminally protected amino acid or peptide. When the amount or concentration of the components is less than the lower limit, the yield of the reaction with the capping agent may decrease. When the amount or concentration of the components exceeds the upper limit, these components may not be sufficiently dissolved in the solvent, resulting in deposition in a flow reactor. The deposition of the components may cause clogging of a flow reactor. By carrying out this step in an amount or concentration in the aforementioned range, it is possible to form an activated derivative with high efficiency and produce a peptide in a short time without clogging of the flow reactor.

In the case of the embodiment, the flow velocity at which the reaction mixture containing the N-terminally and C-terminally protected peptide extended N-terminally is introduced into a flow reactor is preferably in the range of 0.3 to 300 mL/min, and more preferably in the range of 1.5 to 60 mL/min. The flow velocity of the reaction mixture containing the N-terminally and C-terminally protected peptide extended N-terminally can be varied in the above range depending on the flow velocity of each solution in the condensation step. The flow velocity at which the solution containing the capping agent is introduced into a flow reactor is preferably in the range of 0.05 to 50 mL/min, and more preferably in the range of 0.25 to 10 mL/min. When the flow velocity is less than the lower limit, the time required to produce a peptide by the method of the present aspect may be longer. In addition, when the flow velocity exceeds the upper limit, the efficiency of the reaction with the capping agent may decrease. The peptide can be produced in a short time by carrying out this step at a flow velocity in the above range.

In this step, the temperature of the reaction between the unreacted C-terminally activated derivative of the N-terminally protected amino acid or peptide and the capping agent in a flow reactor is preferably in the range of 0 to 100°C, and more preferably in the range of 20 to 60°C. When the temperature of the reaction is less than the lower limit, the efficiency of the reaction may decrease. In addition, when the temperature of the reaction exceeds the upper limit, a by-product may be formed. The desired peptide can be produced in high yield by carrying out this step at a temperature in the above range.

In this step, the time of the reaction between the unreacted C-terminally activated derivative of the N-terminally protected amino acid or peptide and the capping agent in a flow reactor is preferably in the range of 1 second to 60 minutes, and more preferably in the range of 10 to 60 seconds. When the time of the reaction is less than the lower limit, the efficiency of the reaction may decrease. In addition, when the time of the reaction exceeds the upper limit, a by-product may be formed. The desired peptide can be produced in high yield by carrying out this step within the above time range.

By carrying out this step under the above conditions, an unreacted C-terminally activated derivative of the N-terminally protected amino acid or peptide can be inactivated by reacting the unreacted C-terminally activated derivative of the N-terminally protected amino acid or peptide with a capping agent in a flow reactor, whereby the desired peptide can be produced in high yield by substantially suppressing the formation of a by-product caused by the unreacted C-terminal activated derivative of the N-terminally protected amino acid or peptide.

### [1-4. N-terminal Deprotection Step (S4)]

This step includes introducing the N-terminally and C-terminally protected peptide extended N-terminally and a deprotecting agent for an N-terminal protecting group into a flow reactor, thereby removing the N-terminal protecting group for deprotection in the flow reactor to form a C-terminally protected peptide extended N-terminally.

The N-terminally and C-terminally protected peptide extended N-terminally used in this step is obtained in the condensation step or capping step described above.

The deprotecting agent for an N-terminal protecting group used in this step can be appropriately selected according to the N-terminal protecting group of the N-terminally and C-terminally protected peptide extended N-terminally. For example, when the N-terminal protecting group is a group to be removed for deprotection by a base, such as an Fmoc group, the deprotecting agent is preferably an organic base such as 1,8-diazabicyclo[5.4.0]-7-undecene (DBU) or piperidine, and more preferably DBU. When the N-terminal protecting group is a group to be removed for deprotection by an acid, such as a Boc group, the deprotecting agent is preferably an acid such as trifluoroacetic acid (TFA) or hydrogen chloride dissolved in an organic solvent, and more preferably TFA. When the N-terminal protecting group is a group to be removed for deprotection by catalytic reduction, such as a Cbz group, the deprotecting agent is preferably a catalyst, such as palladium on carbon (Pd/C) or Pd(OH)₂/C, and more preferably palladium on carbon. When the N-terminal protecting group is a group to be removed deprotection by nucleophilic substitution reaction using a palladium catalyst, such as an Alloc group, the deprotecting agent is preferably a palladium (0) catalyst, and more preferably tetrakis(triphenylphosphine)palladium (0). By carrying out this step using the deprotecting agent illustrated above, the N-terminal protecting group can be rapidly removed deprotection.

In this step, when a highly reactive deprotection by-product is formed by deprotection reaction of the N-terminal protecting group, the deprotection reaction is preferably carried out in the presence of a scavenger for trapping the deprotection by-product. The scavenger can be appropriately selected according to the N-terminal protecting group of the N-terminally and C-terminally protected peptide extended N-terminally. For example, when the N-terminal protecting group is an Fmoc group, the scavenger is preferably a mercaptoalkane sulfonate or a base such as piperidine capable of trapping dibenzofulvene, which is a deprotection by-product, and more preferably sodium 3-mercapto-1-propanesulfonate (3-MPSNa). When the N-terminal protecting group is a Boc group, the scavenger is preferably anisole, thioanisole, dimethyl sulfide, or thiophenol, for example. By carrying out this step using the deprotecting agent illustrated above, a highly reactive deprotection by-product can be trapped and inactivated, whereby a peptide can be produced in high yield.

In the present embodiment, the scavenger may be introduced into a flow reactor together with or separately from the N-terminally and C-terminally protected peptide extended N-terminally and the deprotecting agent for the N-terminal protecting group. Preferably, the deprotecting agent for the N-terminal protecting group and the scavenger are separately introduced into a flow reactor. More preferably, the solution containing the N-terminally and C-terminally protected peptide extended N-terminally and the solution containing a scavenger are introduced into a flow reactor together or separately, followed by introduction of the solution containing a deprotecting agent for the N-terminal protecting group into the flow reactor. Even more preferably, the solution containing the N-terminally and C-terminally protected peptide extended N-terminally and the solution containing a scavenger are separately introduced into a flow reactor, followed by introduction of the solution containing a deprotecting agent for the N-terminal protecting group into the flow reactor. The deprotection by-product can be trapped efficiently by introducing each solution into a flow reactor in the above order.

The deprotecting agent and scavenger used in this step may be prepared, for example, by purchasing a commercially available product.

This step can be performed by introducing the reaction mixture containing the N-terminally and C-terminally protected peptide extended N-terminally, the solution containing a deprotecting agent in a solvent, and optionally the solution containing a scavenger in a solvent into a flow reactor at a predetermined flow velocity. The solvent can be the same as those illustrated in the description of the C-terminal activation step. The reaction mixture containing the N-terminally and C-terminally protected peptide extended N-terminally usually contains the solvent used in the condensation step or capping step. The solution containing a deprotecting agent and the solution containing a scavenger preferably contain the hydrophilic organic solvent illustrated above as a solvent, and more preferably contain DMSO as a solvent. By using the solvent illustrated above, it is possible to successfully prepare the solution containing a deprotecting agent and the solution containing a scavenger.

The solvent used in this step may be prepared, for example, by purchasing a commercially available product.

In the case of the present embodiment, as the solution containing the N-terminally and C-terminally protected peptide extended N-terminally, the reaction mixture containing the N-terminally and C-terminally protected peptide extended N-terminally obtained in the condensation step or capping step is preferably used as it is; and the reaction mixture containing the N-terminally and C-terminally protected peptide extended N-terminally obtained in the capping step is more preferably used as it is. The reaction mixture containing the N-terminally and C-terminally protected peptide extended N-terminally obtained in the capping step can be used to produce the desired peptide in high yield by substantially suppressing the formation of a by-product caused by the unreacted C-terminal activated derivative of the N-terminally protected amino acid or peptide.

In the case of the present embodiment, in a reaction mixture containing the N-terminally and C-terminally protected peptide extended N-terminally, the concentration of the N-terminally and C-terminally protected peptide extended N-terminally is preferably in the range of 0.01 to 1.0 mol/L, and more preferably in the range of 0.05 to 0.3 mol/L. The concentration of the N-terminally and C-terminally protected peptide extended N-terminally can be varied in the above range depending on the yield of the reaction in the condensation step and capping step. In the solutions containing a deprotecting agent, the amount of the deprotecting agent is preferably in the range of 1 to 100 molar equivalents, and more preferably in the range of 1 to 10 molar equivalents, with respect to the C-terminally protected amino acid or peptide. In the solutions containing a scavenger, the concentration of the scavenger is preferably in the range of 1 to 10 molar equivalents, and more preferably in the range of 1 to 3 molar equivalents, with respect to the C-terminally protected amino acid or peptide. When the amount or concentration of the components is less than the lower limit, the yield of the deprotection reaction may decrease. When the amount or concentration of the components exceeds the upper limit, these components may not be sufficiently dissolved in the solvent, resulting in deposition in the flow reactor. The deposition of the components may cause clogging of a flow reactor. By carrying out this step in an amount or concentration in the above range, it is possible to deprotect the N-terminal protecting group with high efficiency and produce a peptide in a short time without clogging of a flow reactor.

In the case of the embodiment, the flow velocity at which the reaction mixture containing the N-terminally and C-terminally protected peptide extended N-terminally is introduced into a flow reactor is preferably in the range of 0.35 to 350 mL/min, and more preferably in the range of 1.75 to 70 mL/min. The flow velocity of the reaction mixture containing the N-terminally and C-terminally protected peptide extended N-terminally can be varied in the above range depending on the flow velocity of each solution in the condensation step and capping step. The flow velocity at which the solution containing a deprotecting agent is introduced into a flow reactor is preferably in the range of 0.1 to 100 mL/min, and more preferably in the range of 0.5 to 20 mL/min. The flow velocity at which the solution containing a scavenger is introduced into a flow reactor is preferably in the range of 0.05 to 50 mL/min, and more preferably in the range of 0.25 to 10 mL/min. When the flow velocity is less than the lower limit, the time required to produce a peptide by the method of the present aspect may be longer. In addition, when the flow velocity exceeds the upper limit, the yield of the deprotection reaction may decrease. The peptide can be produced in a short time by carrying out this step at a flow velocity in the above range.

In this step, the temperature of the deprotection reaction in a flow reactor is preferably in the range of 0 to 100°C, and more preferably in the range of 5 to 60°C. When the temperature of the deprotection reaction is less than the lower limit, the efficiency of the reaction may decrease. In addition, when the temperature of the deprotection reaction exceeds the upper limit, a by-product may be formed. By carrying out this step at a temperature in the above range, the N-terminal protecting group can be rapidly removed for deprotection to produce a desired peptide in high yield.

In this step, the time of the deprotection reaction in a flow reactor is preferably in the range of 1 second to 60 minutes, and more preferably in the range of 10 to 60 seconds. When the time of the deprotection reaction is less than the lower limit, the efficiency of the reaction may decrease. In addition, when the time of the deprotection reaction exceeds the upper limit, a by-product may be formed. By carrying out this step within the above time range, the N-terminal protecting group can be rapidly removed for deprotection to produce a desired peptide in high yield.

By carrying out this step under the above conditions, it is possible to deprotect the N-terminal protecting group to form the C-terminally protected peptide extended N-terminally in a flow reactor, whereby the N-terminal protecting group can be rapidly removed for deprotection to produce a desired peptide in high yield.

### [1-5. Extraction Step (S5)]

The step includes mixing a reaction mixture containing the C-terminally protected peptide extended N-terminally with an aqueous phase to extract the C-terminally protected peptide extended N-terminally into an organic phase, followed by separation of the organic phase from the aqueous phase.

As described above, the C-terminally protected peptide extended N-terminally has a tag protecting group as a protecting group for the C-terminal carboxyl group that reduces its polarity so that the peptide can be separated and purified by two phase separation of organic and aqueous phases. Therefore, the reaction mixture containing the C-terminally protected peptide extended N-terminally is mixed with an aqueous phase, whereby the mixture is separated into two phases: an organic phase containing the peptide and an aqueous phase. Since the unreacted raw material used in each of the above-described steps and the by-product formed by the reaction are transferred to the aqueous phase, the C-terminally protected peptide extended N-terminally can be extracted by separating the organic phase from the mixture.

The aqueous phase used in this step may contain, in addition to water, if desired, a salt such as sodium chloride, sodium carbonate, potassium hydrogen carbonate, and sodium hydrogen carbonate, or an acid such as hydrochloric acid and sulfuric acid. In addition, the aqueous phase may contain a hydrophilic organic solvent illustrated above, if desired. The inclusion of the salt, acid, and/or hydrophilic organic solvent illustrated above in the aqueous phase can improve the extraction efficiency of the C-terminally protected peptide extended N-terminally.

The salt, acid, and hydrophilic organic solvent used in this step may be prepared, for example, by purchasing a commercially available product.

This step may be performed by a flow synthesis method or by batch processing.

When this step is performed by a flow synthesis method, the reaction mixture containing a C-terminally protected peptide extended N-terminally and the aqueous phase are introduced into a flow reactor at a predetermined flow velocity, and the C-terminally protected peptide extended N-terminally is extracted in the flow reactor. In the case of the present embodiment, the flow velocity at which the reaction mixture containing the C-terminally protected peptide extended N-terminally is introduced into a flow reactor is preferably in the range of 0.4 to 400 mL/min, and more preferably in the range of 2.5 to 100 mL/min. The flow velocity of the reaction mixture containing the C-terminally protected peptides extended N-terminally can be varied in the above range depending on the flow velocity of each solution in the deprotection step. The flow velocity at which the aqueous phase is introduced into a flow reactor is preferably in the range of 0.1 to 1600 mL/min, and more preferably in the range of 1.25 to 200 mL/min. When the flow velocity is less than the lower limit, the time required to produce a peptide by the method of the present aspect may be longer. In addition, when the flow velocity exceeds the upper limit, the extraction efficiency of the C-terminally protected peptide extended N-terminally may decrease. The peptide can be produced in a short time by carrying out this step at a flow velocity in the above range.

In the case of the embodiment, the temperature of extraction in a flow reactor is preferably in the range of 5 to 60°C, and more preferably in the range of 10 to 30°C. When the temperature of extraction is less than the lower limit, the efficiency of extraction may decrease. In addition, when the temperature of extraction exceeds the upper limit, a by-product may be formed. The desired peptide can be produced in high yield by carrying out this step at a temperature in the above range.

In the case of the embodiment, the time of extraction in a flow reactor is preferably in the range of 30 seconds to 24 hours, and more preferably in the range of 30 seconds to 30 minutes. When the time of extraction is less than the lower limit, the efficiency of extraction may decrease. In addition, when the time of extraction exceeds the upper limit, a by-product may be formed. The desired peptide can be produced in high yield by carrying out this step within the above time range.

When this step is performed by batch processing, the reaction mixture containing a C-terminally protected peptide extended N-terminally and the aqueous phase are introduced into a vessel, and the C-terminally protected peptide extended N-terminally is extracted in the vessel.

In the case of the embodiment, the temperature of extraction in a vessel is preferably in the range of 5 to 60°C, and more preferably in the range of 10 to 30°C. When the temperature of extraction is less than the lower limit, the efficiency of extraction may decrease. In addition, when the temperature of extraction exceeds the upper limit, a by-product may be formed. The desired peptide can be produced in high yield by carrying out this step at a temperature in the above range.

In the case of the embodiment, the time of extraction in a vessel is preferably in the range of 0.5 seconds to 24 hours, and more preferably in the range of 1 second to 30 minutes. When the time of extraction is less than the lower limit, the efficiency of extraction may decrease. In addition, when the time of extraction exceeds the upper limit, a by-product may be formed. The desired peptide can be produced in high yield by carrying out this step within the above time range.

The desired peptide can be produced in high yield with a small number of steps in a short time by carrying out this step under the above conditions.

An extract containing the C-terminally protected peptide extended N-terminally obtained in this step can be used as the C-terminally protected amino acid or peptide in the condensation step as it is or in a concentrated state, whereby a peptide chain can be continuously extended to produce a peptide having a desired amino acid sequence.

### [1-6. C-terminal Deprotection Step (S6)]

This step includes reacting the C-terminally protected peptide extended N-terminally obtained in the extraction step with a C-terminal deprotecting agent, thereby removing the C-terminal tag protecting group for deprotection to form a peptide extended N-terminally.

This step can be performed by reacting the extract containing a C-terminally protected peptide extended N-terminally obtained in the extraction step, as it is or in a concentrated state, with a deprotecting agent for the C-terminal tag protecting group and, optionally, a deprotecting agent for the side chain protecting group. In the case of the present embodiment, the deprotecting agent for the C-terminal tag protecting group and the deprotecting agent for the side chain protecting group can be appropriately selected according to the C-terminal tag protecting group and side chain protecting group used.

By carrying out this step under the above conditions, the C-terminal tag protecting group and side chain protecting group can be removed for deprotection to obtain a desired peptide in a free form, whereby the desired peptide can be produced in high yield with a small number of steps in a short time.

### <2. Apparatus for Producing Peptide>

Another aspect of the present invention relates to an apparatus for producing a peptide. By using the apparatus of the present aspect, the desired peptide can be produced in high yield with a small number of steps in a short time.

The apparatus of the present aspect includes:
a C-terminal activation unit comprising a flow reactor and a raw material introducing member for introducing an N-terminally protected amino acid or peptide, in which an N-terminal amino group is protected by an N-terminal protecting group, and a C-terminal activator into the flow reactor, for activating a C-terminal carboxyl group of the N-terminally protected amino acid or peptide in the flow reactor to form a C-terminally activated derivative of the N-terminally protected amino acid or peptide;
a condensation unit comprising a flow reactor and a raw material introducing member for introducing the C-terminally activated derivative of the N-terminally protected amino acid or peptide and a C-terminally protected amino acid or peptide, in which a C-terminal carboxyl group is protected by a tag protecting group, into the flow reactor, for condensing the N-terminally protected amino acid or peptide with the C-terminally protected amino acid or peptide on the N-terminal side in the flow reactor to form an N-terminally and C-terminally protected peptide extended N-terminally;
an N-terminal deprotection unit comprising a flow reactor and a raw material introducing member for introducing the N-terminally and C-terminally protected peptide extended N-terminally and a deprotecting agent for an N-terminal protecting group into the flow reactor, for removing the N-terminal protecting group for deprotection in the flow reactor to form a C-terminally protected peptide extended N-terminally; and
an extraction unit comprising a mixing member for mixing a reaction mixture containing the C-terminally protected peptide extended N-terminally with an aqueous phase, for mixing the reaction mixture containing the C-terminally protected peptide extended N-terminally with the aqueous phase to extract the C-terminally protected peptide extended N-terminally into an organic phase.

The apparatus of the present aspect may further include, if desired, a capping unit comprising a flow reactor and a raw material introducing member for introducing a reaction mixture containing the N-terminally and C-terminally protected peptide extended N-terminally and a capping agent having an amino group into the flow reactor, for reacting the unreacted C-terminally activated derivative of the N-terminally protected amino acid or peptide with the capping agent in the flow reactor to inactivate the C-terminally activated derivative.

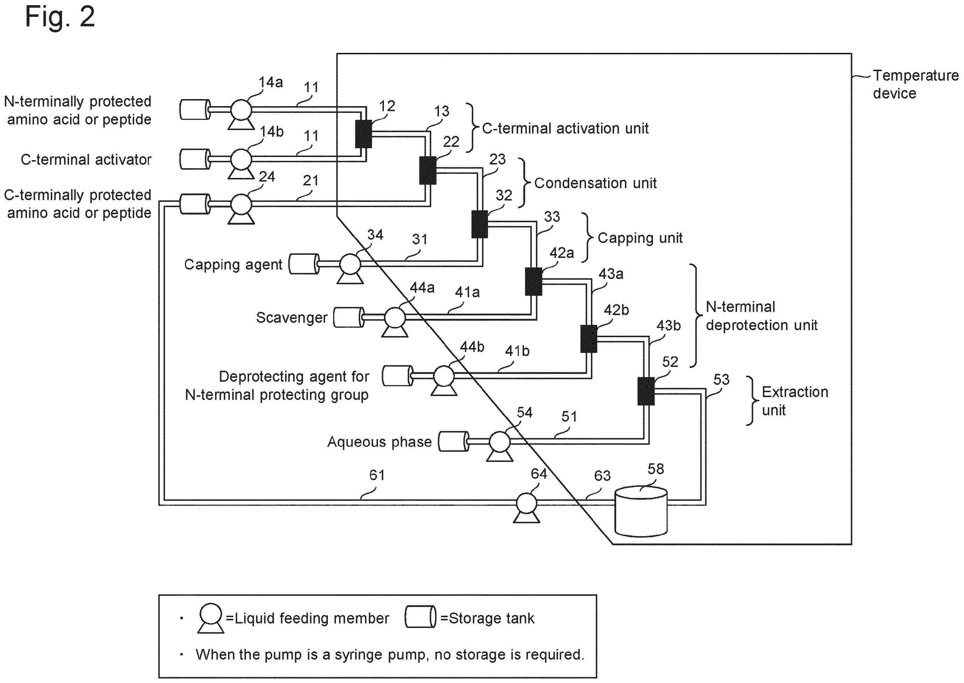

FIG. 2 is a schematic diagram showing one embodiment of the apparatus of the present aspect. As shown in FIG. 2, in the apparatus of the present aspect, the C-terminal activation unit, the condensation unit, the capping unit, and the N-terminal deprotection unit each have a flow reactor and a raw material introducing member. The flow reactor usually comprises two or more inlets 11, 21, 31, 41a, and 41b; mixers 12, 22, 32, 42a, and 42b for mixing a reaction liquid flowing in from the two or more inlets 11, 21, 31, 41a, and 41b; and one or more outlets 13, 23, 33, 43a, and 43b for discharging the reaction liquid mixed in the mixers 12, 22, 32, 42a, and 42b. The inlet and outlet are preferably pipes made of materials to be described below. The raw material introducing member usually comprises at least pumps 14a, 14b, 24, 34, 44a, and 44b for feeding the materials. The pump typically has a storage tank for storing the raw material, a liquid feeding member, and a flow channel for connecting the storage tank and the liquid feeding member. Examples of the pump include a syringe pump, a diaphragm pump, and a high performance liquid chromatography (HPLC) pump. For example, the pump need not have a storage tank as a separate component in the case of a syringe pump because the raw material can be stored in a cylinder of the syringe. In the condensation unit, capping unit, and N-terminal deprotection unit, one or more outlets 13, 23, 33, 43a, and 43b, through which the reaction liquid containing the product obtained in the previous step is flowed out, are preferably used as the raw material introducing member for introducing the product.

In one embodiment, the extraction unit comprises an extraction member, a raw material introducing member, and an aqueous-phase introducing member. The extraction member usually comprises at least two or more inlets 43b and 51, a mixer 52 for mixing a reaction liquid and an aqueous phase flowing in from the two or more inlets 43b and 51, one or more outlets 53 for discharging a mixed phase of the reaction liquid and the aqueous phase mixed in the mixer 52, a phase separating member 58 for separating a mixed phase of the reaction liquid and the aqueous phase flowing in from the one or more outlets 53 into an organic phase and an aqueous phase, and one or more outlets 63 for discharging the organic phase separated by the phase separating member 58. The inlet and outlet are preferably pipes made of a material to be described below. The aqueous-phase introducing member usually comprises at least a pump 54 for feeding the aqueous phase. The pump typically has a storage tank for storing the raw material, a liquid feeding member, and a flow channel for connecting the storage tank and the liquid feeding member. Examples of the pump include a syringe pump, a diaphragm pump, and an HPLC pump. For example, the pump need not have a storage tank as a separate component in the case of a syringe pump because the raw material can be stored in a cylinder of the syringe. In the extraction unit, one or more outlets 43b, through which the reaction liquid containing a product obtained in the previous N-terminal deprotection unit is flowed out, are preferably used as raw material introducing members for introducing the product. In this case, a pressure regulating unit for adjusting the pressure of the effluent is preferably provided in the middle of the outlet 43b from the N-terminal deprotection unit. The extraction unit may have two or more combinations of the extraction member, raw material introducing member, and aqueous-phase introducing member described above.

The apparatus of the present aspect may further include, if desired, a circulating flow unit for circulating and flowing the C-terminally protected peptide extended N-terminally obtained in the extraction unit into the raw material introducing member in the condensation step.

In the present embodiment, the circulating flow unit comprises at least a circulation inlet 61 for circulating and flowing the C-terminally protected peptide extended N-terminally obtained by the extraction unit into the raw material introducing member in the condensation step, and a pump 64 for feeding the C-terminally protected peptide extended N-terminally. The circulation inlet is preferably a pipe made of a material to be described below. The pump typically has a storage tank for storing the C-terminally protected peptide extended N-terminally, a liquid feeding member, and a flow channel for connecting the storage tank and the liquid feeding member. Examples of the pump include a syringe pump, a diaphragm pump, and an HPLC pump. For example, the pump need not have a storage tank as a separate component in the case of a syringe pump because the C-terminally protected peptide extended N-terminally can be stored in a cylinder of the syringe. Since the apparatus of the present aspect comprises the circulating flow unit, the peptide chain can be continuously extended to produce a peptide having a desired amino acid sequence.

In the apparatus of the present aspect, at least one of the C-terminal activation unit, the condensation unit, the capping unit, and the N-terminal deprotection unit, the extraction unit, and the circulating flow unit has a temperature control device for controlling temperatures of the flow reactor, the raw material introducing member, and/or an aqueous-phase introducing member. The temperature control device is preferably used, for example, to adjust the temperatures of an inlet, an outlet, a circulation inlet, a mixer, a phase separating member, a storage tank of a pump, and/or a flow channel of the pump. With the temperature control device, the temperature of the flow reactor, the raw material introducing member, and/or the aqueous-phase introducing member can be adjusted based on physical properties detected by a physical property detecting member described below.

In the apparatus of the present aspect, at least one of the C-terminal activation unit, the condensation unit, the capping unit, and the N-terminal deprotection unit, the extraction unit, and the circulating flow unit comprises at least one physical property detecting member for detecting physical properties of the raw material and the reaction liquid. The physical property detecting member is preferably at least one member selected from the group consisting of a pressure gauge, a flowmeter, and a thermometer. The physical property detecting member is preferably arranged in the middle of the inlet, outlet, and/or circulation inlet. When at least one of the units comprises at least one physical property detecting member, the physical properties of the raw material and the reaction liquid can be detected to confirm the progress of the reaction and adjust the reaction conditions based on the physical properties.

At least one of the C-terminal activation unit, the condensation unit, the capping unit, the N-terminal deprotection unit, the extraction unit, and the circulating flow unit preferably comprises at least one compound detecting member for detecting a compound contained in the raw material and the reaction liquid. The compound detecting member is preferably at least one member selected from the group consisting of an ultraviolet-visible spectroscopy detector, a photodiode array detector, a fluorescence detector, a differential refractive index detector, an electrical conductivity detector, an evaporative light scattering detector, an infrared spectrometer, a near-infrared spectrometer, a nuclear magnetic resonance device, and a mass spectrometer. The compound detecting member is preferably arranged in the middle of the inlet and/or the outlet. In the present embodiment, at least one of the C-terminal activation unit, the condensation unit, the capping unit, and the N-terminal deprotection unit, the extraction unit, and the circulating flow unit preferably further comprises a compound separating member for separating a compound to be detected by the compound detecting member. In this case, the compound separating member is usually arranged at a front part (i.e., upstream side) of the compound detecting member in a flow channel of, for example, the raw material and the reaction liquid. When at least one of the units comprises at least one compound detecting member, the compound contained in the raw material and the reaction liquid can be detected to confirm the progress of the reaction.

At least one of the C-terminal activation unit, the condensation unit, the capping unit, the N-terminal deprotection unit, the extraction unit, and the circulating flow unit preferably comprises at least one outlet port for sampling a reaction liquid, for example. The outlet port is preferably arranged in at least one of the outlets in at least one of the units. When at least one of the units comprises at least one outlet port, the reaction liquid or the like can be sampled to confirm the progress of the reaction.

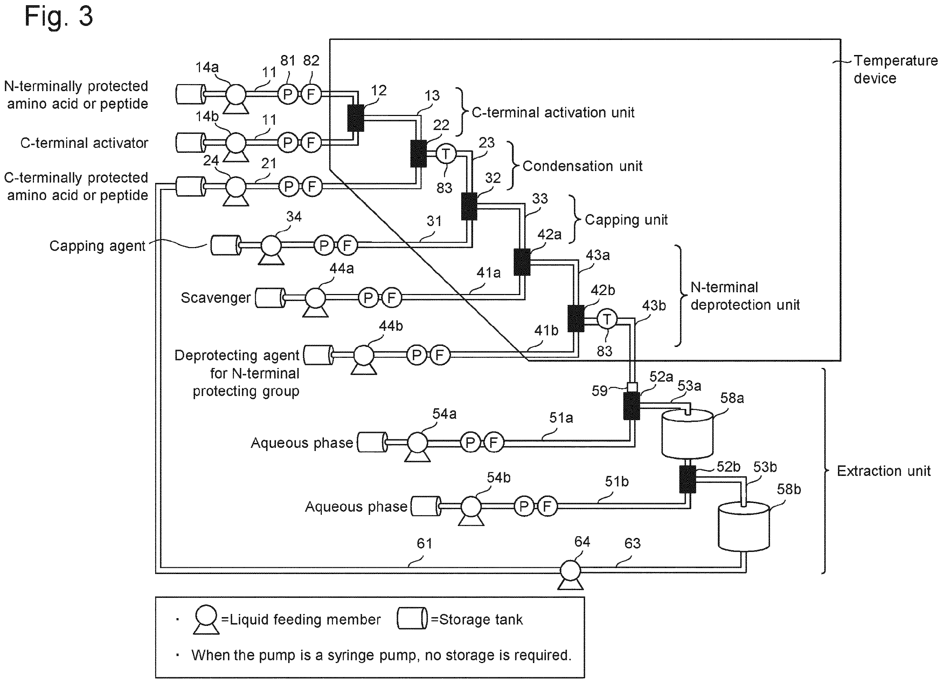

FIG. 3 is a schematic diagram showing another embodiment of the apparatus of the present aspect. As shown in FIG 3, the apparatus of the present embodiment may comprise a C-terminal activation unit, a condensation unit, a capping unit, an N-terminal deprotection unit, a two-step extraction unit, and a circulating flow unit. The C-terminal activation unit comprises at least a pump 14a for feeding an N-terminally protected amino acid or peptide, a pump 14b for feeding a C-terminal activator, two inlets 11 for allowing these raw materials to flow in, a mixer 12 for mixing the reaction liquid flowing in from the two inlets 11, and an outlet 13 for allowing the reaction liquid mixed by the mixer 12 to flow out. The condensation unit comprises at least a pump 24 for feeding a C-terminally protected amino acid or peptide, an inlet 21 for allowing the C-terminally protected amino acid or peptide to flow in, an outlet 13 from the C-terminal activation unit, a mixer 22 for mixing the reaction liquid flowing in from the inlet 21, and an outlet 23 for allowing the reaction liquid mixed by the mixer 22 to flow out. The capping unit comprises at least a pump 34 for feeding the capping agent, an inlet 31 for allowing the capping agent to flow in, an outlet 23 from the condensation unit, a mixer 32 for mixing the reaction liquid flowing in from the inlet 31, and an outlet 33 for allowing the reaction liquid mixed by the mixer 32 to flow out. The N-terminal deprotection unit comprises at least a pump 44b for feeding the deprotection agent for the N-terminal protecting group, an inlet 41b for allowing the deprotection agent for the N-terminal protecting group to flow in, a mixer 42b for mixing the reaction liquid flowing in from the outlet from the capping unit and the inlet 41b, and an outlet 43b for allowing the reaction liquid mixed by the mixer 42b to flow out. The N-terminal deprotection unit preferably comprises, on the upstream side of the mixer 42b, a pump 44a for feeding a scavenger for trapping a deprotection by-product, an inlet 41a for allowing the scavenger to flow in, an outlet 33 from the capping unit, a mixer 42a for mixing the reaction liquid flowing in from the inlet 41a, and an outlet 43a for allowing the reaction liquid mixed by the mixer 42a to flow out. The extraction unit preferably comprises pumps 54a and 54b for feeding the aqueous phase, inlets 51a and 51b for allowing the aqueous phase to flow in, mixers 52a and 52b for mixing the reaction liquid flowing in from the outlet 43b from the N-terminal deprotection unit and the aqueous phase flowing in from the inlets 51a and 51b, outlets 53a and 53b for allowing the mixed phase of the reaction liquid and the aqueous phase mixed in the mixers 52a and 52b to flow out, phase separating members 58a and 58b for separating the mixed phase of the reaction liquid and the aqueous phase mixed in the mixers 52a and 52b into an organic phase and an aqueous phase, and one or more outlets 63 for allowing the organic phase separated by the phase separating members 58a and 58b to flow out. In addition, the extraction unit preferably comprises a pressure regulating unit 59 for adjusting the pressure of the effluent in the middle of the outlet 43b from the N-terminal deprotection unit. The circulating flow unit comprises at least a circulation inlet 61 for circulating and flowing the C-terminally protected peptide extended N-terminally obtained by the extraction unit into the raw material introducing member in the condensation step, and a pump 64 for feeding the C-terminally protected peptide extended N-terminally. The C-terminal activation unit, the condensation unit, the capping unit, and the N-terminal deprotection unit preferably comprise a temperature control device for controlling temperatures of the flow reactor, raw material introducing member, and/or the aqueous-phase introduction member. Preferably, at least one pressure gauge 81 and at least one flowmeter 82 are provided in the middle of the inlet of the C-terminal activation unit, condensation unit, capping unit, N-terminal deprotection unit, and extraction unit. Preferably, at least one thermometer 83 is provided in the middle of the outlet of the condensation unit and the outlet of the N-terminal deprotection unit.

In the apparatus of the present aspect, examples of the materials of the respective members constituting the flow reactor and raw material introducing member include, but are not limited to, metals, fluorine resins (e.g., polytetrafluoroethylene (PTFE), perfluoro alkoxyl alkane (PFA), ethylene tetrafluoroethylene copolymer (ETFE), and perfluoroethylene propene copolymer (FEP)), polyetheretherketone (PEEK), polyethylene (PE), polypropylene (PP), glass, and silicon. By using the flow reactor and raw material introducing member composed of the above materials, the influence of a solvent or the like can be substantially avoided. The inlet and outlet are preferably tubes of the above materials commonly used in the prior art, and more preferably PTFE tubes. Preferably, the mixer, syringe, and pump are of the aforementioned materials commonly used in the prior art. The inner diameters of the inlet, outlet, and mixer are preferably 5 mm or less and more preferably in the range of 0.25 to 2 mm. By using a flow reactor composed of a member having an inner diameter in the above range, the apparatus of the present embodiment can be applied to the method for producing a peptide according to one aspect of the present invention, which is carried out in a microflow system.

In the apparatus of the present aspect, the extraction unit comprises a mixing member for mixing the reaction mixture containing a C-terminally protected peptide extended N-terminally with the aqueous phase. The mixing member can be appropriately selected according to an aspect of the extraction. For example, when the extraction is performed by a flow synthesis method, the above-described flow reactor can be used as the mixing member. Alternatively, when the extraction is performed by batch processing, vessels having various shapes made of glass, metal, or plastic commonly used in the prior art can be used as mixing members.

As described in detail above, the method and apparatus for producing a peptide of one aspect of the present invention can produce a desired peptide in a high yield with a small number of steps in a short time as compared with the method and apparatus of the prior art.

### Examples

Hereinafter, the present invention is described in more detail with reference to Examples, but the technical scope of the present invention is not limited to these Examples.

### <I: Apparatus for Producing Peptide>

As shown in FIG. 2, an apparatus for producing a peptide with a plurality of flow reactors (hereinafter, also referred to as "production apparatus 1") was produced using a polytetrafluoroethylene (PTFE) tube (inner diameter: 0.8 mm, outer diameter: 1.59 mm), a T-shaped mixer (inner diameter: 0.25 mm, manufactured by Sankoh Seiki Kogyo Co., Ltd.), a syringe pump (PHD ULTRA, manufactured by Harvard Apparatus), and a syringe having a volume of 10 to 50 ml (SGE syringe, manufactured by Trajan Scientific and Medical).

As shown in FIG. 3, an apparatus for producing a peptide with a plurality of flow reactors (hereinafter also referred to as "production apparatus 2") was produced using a polytetrafluoroethylene (PTFE) tube (inner diameter: 1.59 mm, outer diameter: 1/8 inch), a T-shaped mixer (inner diameter: 0.5 mm, manufactured by IDEX), a Y-shaped mixer (inner diameter: 1.5 mm, manufactured by IDEX), an HPLC pump (PU-4086, manufactured by JASCO Corporation), a diaphragm pump (QI-30, manufactured by TACMINA CORPORATION), and a back pressure regulator (BPR-1000, manufactured by Zaiput Flow Technologies). A flowmeter and a pressure gauge were installed in the flow channel between the pump feeding each reagent and the T-shaped mixer to measure a flow rate and pressure during the synthesis. A thermometer was installed in the outlet of the condensation unit and the outlet of the N-terminal deprotection unit to measure the temperature of the reaction solution. In the present apparatus, at least one compound detecting member such as a near-infrared spectrometer can be installed in the capping unit and/or the N-terminal deprotection unit to detect disappearance of the raw material and/or formation of the reaction product. The apparatus can also be provided with a loop and a valve for sampling the reaction liquid and the like in the outlet of the capping unit and/or outlet of the N-terminal deprotection unit to sample and analyze each reaction liquid and the like.

### <II: Production of Peptide by Method of the Present Invention>

### [II-1: Synthesis of C-terminally Protected Amino Acid]

According to the method described in International Publication No. WO 2017/038650, a C-terminal carboxyl group of Fmoc-Arg(Pbf)-OH was protected by a tag protecting group of a monovalent group (a radical obtained by eliminating a hydrogen atom from a hydroxyl group) derived from a compound (TIPS2-OH) represented by the following formula to synthesize H-Arg(Pbf)-O-TIPS2 as a C-terminally protected amino acid.

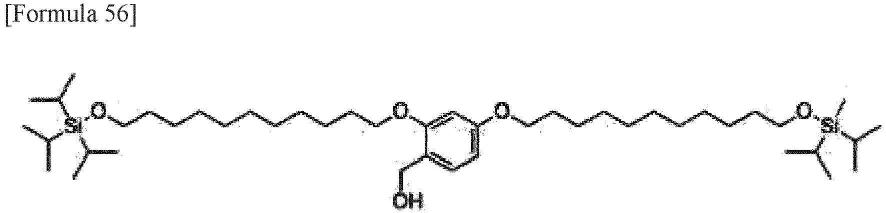

### [II-2: Synthesis of H-Arg(Pbf)-Arg(Pbf)-O-TIPS2 (Example 1)]

The following reaction was carried out using the production apparatus 1. A solution 1 (DMF solution containing 0.433 mol/L of Fmoc-Arg(Pbf)-OH, 0.043 mol/L of N,N,-dimethylbenzylamine (DMBA), and 0.433 mol/L of DIEA) and a solution 2 (cyclopentylmethyl ether (CPME) solution containing 0.255 mol/L of IBCF) were fed at a flow velocity of 1.2 mL/min and a flow velocity of 2.0 mL/min, respectively, mixed using a T-shaped mixer, and reacted in a PTFE tube for 5 seconds to form a C-terminally activated derivative of an N-terminally protected amino acid (C-terminal activation step). Subsequently, a solution containing 0.16 mol/L of this C-terminally activated derivative and a solution 3 (mixed solution of DMF and CPME containing 0.200 mol/L of H-Arg(Pbf)-O-TIPS2) were fed at a flow velocity of 3.2 mL/min and a flow velocity of 2.0 mL/min, respectively, mixed using a T-shaped mixer, and reacted in a PTFE tube for 30 seconds to perform a condensation reaction (condensation step). Next, the condensation reaction solution and a solution 4 (DMSO solution containing 1.04 mol/L of AEE) were fed at a flow velocity of 5.2 mL/min and a flow velocity of 0.5 mL/min, respectively, mixed using a T-shaped mixer, and reacted in a PTFE tube for 30 seconds to perform a capping reaction (capping step). Then, the capping reaction solution and a solution 5 (DMSO solution containing 0.81 mol/L of 3-MPSNa) were fed at a flow velocity of 5.7 mL/min and a flow velocity of 1.2 mL/min, respectively, and mixed using a T-shaped mixer. Thereafter, this mixture and a solution 6 (DMSO solution containing 4.86 mol/L of DBU) were further fed at a flow velocity of 6.9 mL/min and a flow velocity of 0.5 mL/min, respectively, mixed using a T-shaped mixer, and reacted in a PTFE tube for 30 seconds to deprotect the Fmoc group (N-terminal deprotection step). The reaction solution flowing out of the PTFE tube was sampled and analyzed by LCMS (LCMS analysis result: m/z = 1611). The process from the C-terminal activation step to the N-terminal deprotection step was performed at 60°C.

### [II-3: Synthesis of H-Phe-Arg(Pbf)-O-TIPS2 (Example 2)]

The following reaction was carried out using the production apparatus 1. A solution 1 (DMF solution containing 0.433 mol/L of Fmoc-Phe-OH, 0.043 mol/L of DMBA, and 0.433 mol/L of DIEA) and a solution 2 (CPME solution containing 0.255 mol/L of IBCF) were fed at a flow velocity of 1.2 mL/min and a flow velocity of 2.0 mL/min, respectively, mixed using a T-shaped mixer, and reacted in a PTFE tube for 5 seconds to form a C-terminally activated derivative of an N-terminally protected amino acid (C-terminal activation step). Subsequently, a solution containing 0.16 mol/L of this C-terminally activated derivative and a solution 3 (mixed solution of DMF and CPME containing 0.200 mol/L of H-Arg(Pbf)-O-TIPS2) were fed at a flow velocity of 3.2 mL/min and a flow velocity of 2.0 mL/min, respectively, mixed using a T-shaped mixer, and reacted in a PTFE tube for 30 seconds to perform a condensation reaction (condensation step). Next, the condensation reaction solution and a solution 4 (DMSO solution containing 1.04 mol/L of AEE) were fed at a flow velocity of 5.2 mL/min and a flow velocity of 0.5 mL/min, respectively, mixed using a T-shaped mixer, and reacted in a PTFE tube for 30 seconds to perform a capping reaction (capping step). Then, the capping reaction solution and a solution 5 (DMSO solution containing 0.81 mol/L of 3-MPSNa) were fed at a flow velocity of 5.7 mL/min and a flow velocity of 1.2 mL/min, respectively, and mixed using a T-shaped mixer. Thereafter, this mixture and a solution 6 (DMSO solution containing 4.86 mol/L of DBU) were further fed at a flow velocity of 6.9 mL/min and a flow velocity of 0.5 mL/min, respectively, mixed using a T-shaped mixer, and reacted in a PTFE tube for 30 seconds to deprotect the Fmoc group (N-terminal deprotection step). The process from the C-terminal activation step to the N-terminal deprotection step was performed at 40°C. The reaction solution of the N-terminal deprotection step was washed with an aqueous sodium carbonate solution after the reaction was terminated with an aqueous sulfuric acid solution, thereby recovering an organic phase (extraction step). The recovered organic phase was dried over sodium sulfate, and then the solvent was distilled off to give 0.8095 g of H-Phe-Arg(Pbf)-O-TIPS2 (yield: quantitative, m/z = 1,350).

### [II-4: Synthesis of H-Ser(tBu)-Phe-Arg(Pbf)-O-TIPS2 (Example 3)]

### (1) Synthesis of H-Phe-Arg(Pbf)-O-TIPS2

Using the production apparatus 1, 3.2491 g of H-Phe-Arg(Pbf)-O-TIPS2 (yield: quantitative) was obtained by performing the synthesis reaction in the same manner as in the procedure described in II-3 except that the reaction time of the N-terminal deprotection step and the reaction temperature from the C-terminal activation step to the N-terminal deprotection step were changed to 10 seconds and 60°C, respectively.

### (2) Synthesis of H-Ser(tBu)-Phe-Arg(Pbf)-O-TIPS2

A solution 1 (DMF solution containing 0.433 mol/L of Fmoc-Ser(tBu)-OH, 0.043 mol/L of DMBA, and 0.433 mol/L of DIEA) and a solution 2 (CPME solution containing 0.255 mol/L of IBCF) were fed at a flow velocity of 1.2 mL/min and a flow velocity of 2.0 mL/min, respectively, mixed using a T-shaped mixer, and reacted in a PTFE tube for 5 seconds to form a C-terminally activated derivative of an N-terminally protected amino acid (C-terminal activation step). Subsequently, a solution containing the C-terminally activated derivative and a solution 3 (mixed solution of DMF and CPME containing 0.200 mol/L of H-Phe-Arg(Pbf)-O-TIPS2 synthesized by the procedure described in (1)) were fed at a flow velocity of 3.2 mL/min and a flow velocity of 2.0 mL/min, respectively, mixed using a T-shaped mixer, and reacted in a PTFE tube for 30 seconds to perform a condensation reaction (condensation step). Next, the condensation reaction solution and a solution 4 (DMSO solution containing 1.04 mol/L of AEE) were fed at a flow velocity of 5.2 mL/min and a flow velocity of 0.5 mL/min, respectively, mixed using a T-shaped mixer, and reacted in a PTFE tube for 30 seconds to perform a capping reaction (capping step). Then, the capping reaction solution and a solution 5 (DMSO solution containing 0.81 mol/L of 3-MPSNa) were fed at a flow velocity of 5.7 mL/min and a flow velocity of 1.2 mL/min, respectively, and mixed using a T-shaped mixer. Thereafter, this mixture and a solution 6 (DMSO solution containing 4.86 mol/L of DBU) were further fed at a flow velocity of 6.9 mL/min and a flow velocity of 0.5 mL/min, respectively, mixed using a T-shaped mixer, and reacted in a PTFE tube for 30 seconds to deprotect the Fmoc group (N-terminal deprotection step). The process from the C-terminal activation step to the N-terminal deprotection step was performed at 60°C. The reaction solution of the N-terminal deprotection step was washed with an aqueous sodium carbonate solution after the reaction was terminated with an aqueous sulfuric acid solution, thereby recovering an organic phase (extraction step). The recovered organic phase was dried over sodium sulfate, and then the solvent was distilled off to give H-Ser(tBu)-Phe-Arg(Pbf)-O-TIPS2. The completion of the reaction was confirmed by observing loss of the raw material and formation of the target product using UPLC.

### [II-5: Synthesis of H-Glu(OtBu)-Ala-Glu(OtBu)-Lys(Boc)-Leu-O-TIPS2 (Example 4)]

### (1) Synthesis of H-Glu(OtBu)-Lys(Boc)-Leu-O-TIPS2

The following reaction was carried out using the production apparatus 1. A solution 1 (DMF solution containing 0.433 mol/L of Fmoc-Glu(OtBu)-OH, 0.043 mol/L of DMBA, and 0.433 mol/L of DIEA) and a solution 2 (CPME solution containing 0.255 mol/L of IBCF) were fed at a flow velocity of 1.2 mL/min and a flow velocity of 2.0 mL/min, respectively, mixed using a T-shaped mixer, and reacted in a PTFE tube for 5 seconds to form a C-terminally activated derivative of an N-terminally protected amino acid (C-terminal activation step). Subsequently, a solution containing 0.16 mol/L of this C-terminally activated derivative and a solution 3 (mixed solution of DMF and CPME containing 0.200 mol/L of H-Lys(Boc)-Leu-O-TIPS2) were fed at a flow velocity of 3.2 mL/min and a flow velocity of 2.0 mL/min, respectively, mixed using a T-shaped mixer, and reacted in a PTFE tube for 30 seconds to perform a condensation reaction (condensation step). Next, the condensation reaction solution and a solution 4 (DMSO solution containing 0.65 mol/L of AEE) were fed at a flow velocity of 5.2 mL/min and a flow velocity of 0.8 mL/min, respectively, mixed using a T-shaped mixer, and reacted in a PTFE tube for 30 seconds to perform a capping reaction (capping step). Then, the capping reaction solution and a solution 5 (DMSO solution containing 0.46 mol/L of 3-MPSNa and 1.16 mol/L of DBU) were fed at a flow velocity of 6.0 mL/min and a flow velocity of 2.1 mL/min, respectively, mixed using a T-shaped mixer, and reacted in a PTFE tube for 30 seconds to deprotect the Fmoc group (N-terminal deprotection step). The process from the C-terminal activation step to the N-terminal deprotection step was performed at 40°C. The reaction solution of the N-terminal deprotection step was washed with an aqueous sodium carbonate solution after the reaction was terminated with an aqueous sulfuric acid solution, thereby recovering an organic phase (extraction step). The recovered organic phase was dried over sodium sulfate, and then the solvent was distilled off to give H-Glu(OtBu)-Lys(Boc)-Leu-O-TIPS2 (yield: quantitative, m/z = 1320).

### (2) Synthesis of H-Ala-Glu(OtBu)-Lys(Boc)-Leu-O-TIPS2

H-Ala-Glu(OtBu)-Lys(Boc)-Leu-O-TIPS2 (yield: 95%, m/z = 1391) was obtained by performing the synthesis reaction in the same manner as in the procedure described in II-5 (1) except that the amino acid contained in the solvent 1 and the peptide contained in the solvent 3 were changed to Fmoc-Ala-OH and H-Glu(OtBu)-Lys(Boc)-Leu-O-TIPS2 obtained in the synthesis described in II-5 (1), respectively.

### (3) Synthesis of H-Glu(OtBu)-Ala-Glu(OtBu)-Lys(Boc)-Leu-O-TIPS2

H-Glu(OtBu)-Ala-Glu(OtBu)-Lys(Boc)-Leu-O-TIPS2 (yield: 96%, m/z = 1576) was obtained by performing the synthesis reaction in the same manner as in the procedure described in II-5 (1) except that the amino acid contained in the solvent 1 and the peptide contained in the solvent 3 were changed to Fmoc-Glu(OtBu)-OH and H-Ala-Glu(OtBu)-Lys(Boc)-Leu-O-TIPS2 obtained in the synthesis described in II-5 (2), respectively.

### [II-6: Synthesis of H-D-Tic-Oic-Arg(Pbf)-O-TIPS2 (Example 5)]

### (1) Synthesis of H-Oic-Arg(Pbf)-O-TIPS2

The following reaction was carried out using the production apparatus 2. A solution 1 (5.882 mol/L of DIEA) and a solution 2 (DMF solution containing 0.520 mol/L of Fmoc-Oic-OH) were fed at a flow velocity of 0.584 mL/min and a flow velocity of 3.0 mL/min, respectively, mixed using a T-shaped mixer, and then mixed with a solution 3 (DMF solution containing 1.0 mol/L of COMU) fed at a flow velocity of 1.5 mL/min using a T-shaped mixer, followed by reaction in a PTFE tube for 5 seconds to form a C-terminally activated derivative of an N-terminally protected amino acid (C-terminal activation step). Subsequently, a solution containing 0.295 mol/L of this C-terminally activated derivative and a solution 4 (mixed solution of DFM and MTHP containing 0.1 mmol/L of H-Arg(Pbf)-O-TIPS2) were fed at a flow velocity of 5.084 mL/min and a flow velocity of 12.0 mL/min, respectively, mixed using a T-shaped mixer, and then reacted in a PTFE tube for 60 seconds to perform a condensation reaction (condensation step). Next, the condensation reaction solution and a solution 5 (DMSO solution containing 0.60 mol/L of AEE) were fed at a flow velocity of 17.084 mL/min and a flow velocity of 2.4 mL/min, respectively, mixed using a T-shaped mixer, and reacted in a PTFE tube for 30 seconds to perform a capping reaction (capping step). Then, the capping reaction solution and a solution 6 (DMSO solution containing 0.446 mol/L of 3-MPSNa and 1.114 mol/L of DBU) were fed at a flow velocity of 19.484 mL/min and a flow velocity of 6.3 mL/min, respectively, mixed using a T-shaped mixer, and reacted in a PTFE tube for 60 seconds to deprotect the Fmoc group (N-terminal deprotection step). Subsequently, the N-terminal deprotection reaction solution and a solution 7 (5% KHCO3 aqueous solution) were fed at a flow velocity of 25.785 mL/min and at a flow velocity of 25.8 mL/min, respectively, and mixed using a Y-shaped mixer. The mixture was further mixed in a PTFE tube for 1 second, followed by separation of an organic phase and an aqueous phase in a settler (extraction step 1). Subsequently, the organic phase was mixed with a solution 8 (20% NaCl aqueous solution) fed at a flow velocity of 12.0 mL/min using a Y-shaped mixer. The mixture was further mixed for 1 second in a PTFF tube, followed by separation of the organic phase and the aqueous phase in a settler (extraction step 2). The process from the C-terminal activation step to the N-terminal deprotection step was performed at 40°C, while the extraction step was performed at room temperature. The completion of the reaction was confirmed by observing loss of the raw material and formation of the target product using UPLC.

### (2) Synthesis of H-D-Tic-Oic-Arg(Pbf)-O-TIPS2

H-D-Tic-Oic-Arg(Pbf)-O-TIPS2 (yield: quantitative) was obtained by performing the synthesis reaction in the same manner as in the procedure described in II-6 (1) except that the amino acid contained in the solvent 2 and the peptide contained in the solvent 4 were changed to Fmoc-D-Tic-OH and H-Oic-Arg(Pbf)-O-TIPS2 obtained in the synthesis described in II-6 (1), respectively, using the production apparatus 2. It was confirmed by mass spectrometer that the target product was obtained.

### <III: Comparison between Production of Peptide by Method of the Present Invention and Production of Peptide by Prior Art Method>

### [III-1: Synthesis of H-Gly-Glu(OtBu)-O-TIPS2 by Method of the Present Invention (Example 6)]

The following reaction was carried out using the production apparatus 1. A solution 1 (DMF solution containing 0.433 mol/L of Fmoc-Gly-OH, 0.043 mol/L of DMBA, and 0.433 mol/L of DIEA) and a solution 2 (CPME solution containing 0.255 mol/L of IBCF) were fed at a flow velocity of 1.2 mL/min and a flow velocity of 2.0 mL/min, respectively, mixed using a T-shaped mixer, and reacted in a PTFE tube for 5 seconds to form a C-terminally activated derivative of an N-terminally protected amino acid (C-terminal activation step). Subsequently, a solution containing 0.16 mol/L of this C-terminally activated derivative and a solution 3 (mixed solution of DMF and CPME containing 0.200 mol/L of H-Glu(OtBu)-O-TIPS2) were fed at a flow velocity of 3.2 mL/min and a flow velocity of 2.0 mL/min, respectively, mixed using a T-shaped mixer, and reacted in a PTFE tube for 30 seconds to perform a condensation reaction (condensation step). Next, the condensation reaction solution and a solution 4 (DMSO solution containing 0.65 mol/L of AEE) were fed at a flow velocity of 5.2 mL/min and a flow velocity of 0.8 mL/min, respectively, mixed using a T-shaped mixer, and reacted in a PTFE tube for 30 seconds to perform a capping reaction (capping step). Then, the capping reaction solution and a solution 5 (DMSO solution containing 0.46 mol/L of 3-MPSNa and 1.16 mol/L of DBU) were fed at a flow velocity of 6.0 mL/min and a flow velocity of 2.1 mL/min, respectively, mixed using a T-shaped mixer, and reacted in a PTFE tube for 30 seconds to deprotect the Fmoc group (N-terminal deprotection step). The process from the C-terminal activation step to the N-terminal deprotection step was performed at 40°C. The reaction solution of the N-terminal deprotection step was washed with an aqueous sodium carbonate solution after the reaction was terminated with an aqueous sulfuric acid solution, thereby recovering an organic phase (extraction step). The recovered organic phase was dried over sodium sulfate, and then the solvent was distilled off to give H-Glu(OtBu)-Lys(Boc)-Leu-O-TIPS2.

### [III-2: Synthesis of H-Gly-Glu(OtBu)-O-TIPS2 by Prior Art Method (Comparative Example 1)]

As a comparative example, a peptide was synthesized in the same manner as in Example 9 described in International Publication No. WO 2020/218497.

### (1) Condensation Reaction and Extraction after Condensation Reaction

A chloroform solution (solution C1) containing 0.06 mmol/mL of H-Glu(OtBu)-O-TIPS2 and a DMF solution (solution C2) containing 0.21 mmol/mL of Fmoc-Gly-OH and 0.05 mmol/mL of HOBt were fed using a syringe pump at a flow velocity of 0.500 mL/min and 0.21 mL/min, respectively, and mixed using a T-shaped mixer (Union Tee SS-100-3, outer diameter: 1/16 inch). A chloroform solution (solution C3) containing 0.32 mmol/mL EDC·HCl was fed at a flow velocity of 0.13 mL/min, mixed with a mixture of the solutions C1 and C2 using a T-shaped mixer (Union Tee SS-100-3, outer diameter: 1/16 inch), and reacted in a PFA tube (inner diameter: 1.0 mm, length 10.0 m) for 9 minutes. Subsequently, 20% by mass of NaCl aqueous solution (solution C4) was fed at a flow velocity of 0.56 mL/min using a syringe pump and mixed with a mixture of the solutions C1, C2 and C3 using a T-shaped mixer (Union Tee SS-200-3, outer diameter: 1/8 inch). The mixed solution was passed through a PFA tube (inner diameter: 1.6 mm, length: 5.0 m) for 7 minutes. The solution after passing through was separated with a separating funnel to recover an organic phase containing Fmoc-Gly-Glu(OtBu)-O-TIPS2.

### (2) Deprotection reaction of Fmoc Group and Washing of Organic Phase with Aqueous Sodium Carbonate Solution after Deprotection Reaction

The solution containing 0.05 mmol/ml of Fmoc-Gly-Glu(OtBu)-O-TIPS2 obtained by the procedure described in III-2 (1) (solution C1) and a DMF solution containing 0.52 mmol/mL of thiomalic acid and 1.57 mmol/mL of DBU (solution C2) were fed using a syringe pump at a flow velocity of 0.73 mL/min and 0.35 mL/min, respectively, mixed using a T-shaped mixer (Union Tee SS-100-3, outer diameter: 1/16 inch), and then reacted in a PFA tube (inner diameter: 1.0 mm, length: 10.0 m) for 7 minutes. To the deprotection reaction solution, 5.0% by mass of Na₂CO₃ aqueous solution (solution C3) was fed at a flow velocity of 0.80 mL/min using a syringe pump, which was then mixed using a T-shaped mixer (Union Tee SS-100-3, outer diameter: 1/16 inch). The mixed solution was passed through a PFA tube (inner diameter: 1.6 mm, length: 5.0 m) for 5 minutes. The solution after passing through was separated with a separating funnel to recover an organic phase.

### (3) Washing of Organic Phase with Aqueous Sodium Chloride Solution after Washing with Aqueous Sodium Carbonate Solution

The organic phase obtained by the procedure described in III-2 (2) (solution C1) and a solution of 20.0 % by mass of an aqueous solution of NaCl and DMF mixed at a volume ratio of 6:4 (solution C2) were each fed at a flow velocity of 1.50 mL/min using a syringe pump, mixed using a T-shaped mixer (Union Tee SS-200-3, outer diameter: 1/8 inch), and then passed through a PFA tube (inner diameter: 1.6 mm, length: 5.0 m) for 3 minutes. The solution after passing through was separated with a separating funnel to recover an organic phase containing H-Gly-Glu(OtBu)-O-TIPS2.

### [III-3: Comparison of Reaction Time between Synthesis by Method of the Present Invention and Synthesis by Prior Art Method Method]

Table 1 shows results of comparing each reaction time in the synthesis by the method of the present invention described in III-1 (Example 5) with each reaction time in the synthesis by the prior art method described in III-2 (Example 9 described in International Publication No. WO 2020/218497) (Comparative Example 1).

**[Table 1]**

| | Example 6 | Comparative Example 1 |
|---|---|---|
| Time required for condensation reaction | 30 seconds | 9 minutes |
| Time required for deprotection reaction | 30 seconds | 7 minutes |

As shown in Table 1, the time required for the condensation reaction and the time required for the deprotection reaction were both 30 seconds in the synthesis by the method of the present invention (Example 6), whereas the time required for the condensation reaction and the time required for the deprotection reaction were both several minutes in the synthesis by the prior art method (Comparative Example 1). In Comparative Example 1, the extraction and liquid separation treatments are carried out during the condensation and deprotection reactions, thus requiring additional time to carry out the entire synthesis process to obtain the target product.

Note that the present invention includes various modifications without being limited to Examples above. For examples, Examples above are described in detail to explain the present invention in an easy-to-understand manner, and the present invention is not always limited to those including all the configurations described above. The configuration of each Example can be partially provided with other configurations, omitted and/or replaced.

### DESCRIPTION OF SYMBOLS

S1 C-terminal activation step
S2 Condensation step
S3 Capping step
S4 N-terminal deprotection step
S5 Extraction step
S6 C-terminal deprotection step
11, 21, 31, 41a, 41b, 51, 51a, 51b Inlet
12, 22, 32, 42a, 42b, 52, 52a, 52b Mixer
13, 23, 33, 43a, 43b, 53, 53a, 53b, 63 Outlet
14a, 14b, 24, 34, 44a, 44b, 54, 54a, 54b, 64 Pump
58, 58a, 58b Phase separating member
59 Pressure regulating unit
61 Circulation inlet
81 Pressure gauge
82 Flowmeter
83 Thermometer

## Claims

1. A method for producing a peptide, comprising:
a C-terminal activation step of introducing an N-terminally protected amino acid or peptide, in which an N-terminal amino group is protected by an N-terminal protecting group, and a C-terminal activator into a flow reactor, thereby activating a C-terminal carboxyl group of the N-terminally protected amino acid or peptide in the flow reactor to form a C-terminally activated derivative of the N-terminally protected amino acid or peptide;
a condensation step of introducing the C-terminally activated derivative of the N-terminally protected amino acid or peptide and a C-terminally protected amino acid or peptide, in which a C-terminal carboxyl group is protected by a tag protecting group, into a flow reactor, thereby condensing the N-terminally protected amino acid or peptide with the C-terminally protected amino acid or peptide on the N-terminal side in the flow reactor to form an N-terminally and C-terminally protected peptide extended N-terminally;
an N-terminal deprotection step of introducing the N-terminally and C-terminally protected peptide extended N-terminally and a deprotecting agent for an N-terminal protecting group into a flow reactor, thereby removing the N-terminal protecting group for deprotection in the flow reactor to form a C-terminally protected peptide extended N-terminally; and
an extraction step of mixing a reaction mixture containing the C-terminally protected peptide extended N-terminally with an aqueous phase to extract the C-terminally protected peptide extended N-terminally into an organic phase.

2. The method according to claim 1, wherein the C-terminal activator is selected from the group consisting of halogenated ethyl formate, halogenated isopropyl formate, halogenated isobutyl formate, a carboxylic acid halide, phosgene and a phosgene equivalent, a carbodiimide condensing agent, a phosphonium condensing agent, an uronium condensing agent, a halouronium condensing agent, an imidazole condensing agent, a triazine condensing agent, and a condensation additive.

3. The method according to claim 1, wherein the tag protecting group is a monovalent group derived from a compound selected from the group consisting of:
(i) a fluorene compound represented by formula (I): 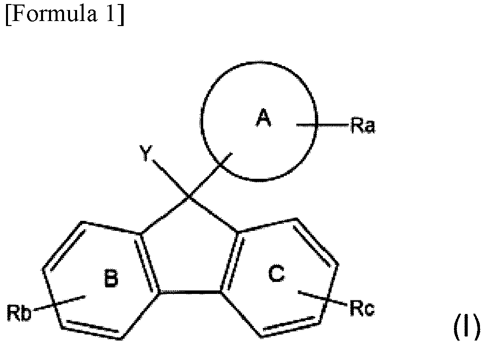 wherein
Ring A represents an aromatic ring; Y is a hydroxyl group, a bromo group, or a chloro group; Ra, Rb, and Rc each independently represent an organic group having an aliphatic hydrocarbon group, a hydrogen atom, or an electron withdrawing group, and at least one of Ra, Rb, and Rc is an organic group having an aliphatic hydrocarbon group; and Rings A, B and C each independently optionally have an electron withdrawing group,
wherein the organic group having an aliphatic hydrocarbon group is at least one group selected from the group consisting of
a group represented by formula (b): 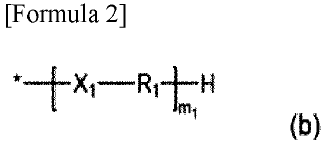 wherein
* represents a binding position; X₁ is -O-; R₁ is an aliphatic hydrocarbon group having 5 to 60 carbon atoms; and m₁ is 1,
a group represented by formula (c): 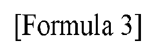 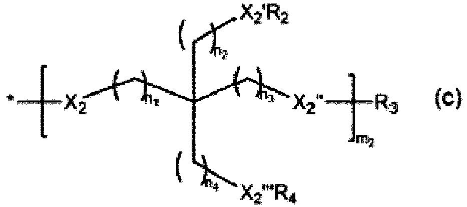 wherein
* represents a binding position; X₂, X₂', X₂", and X₂‴ are -O-; R₂ and R₄ are each independently an aliphatic hydrocarbon group having 5 to 60 carbon atoms; R₃ is an organic group having an aliphatic hydrocarbon group having 5 to 60 carbon atoms; n₁, n₂, n₃, and n₄ are 1; and m₂ is 1, and
a group represented by formula (d): 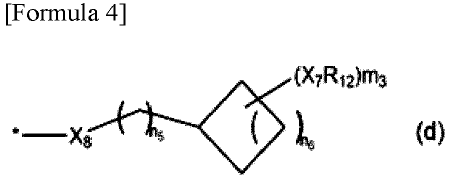 wherein
* represents a binding position; X₈ represents -O-; m₃ is 2 or 3; n₅ is 1; n₆ is 3; X₇ is -O-; m₃ number of R₁₂ are each independently an alkyl group having 4 to 30 carbon atoms,
wherein the organic group having the aliphatic hydrocarbon group is present at a 2- and/or 7-position of the fluorene compound,
(ii) a fluorene compound represented by formula (II): 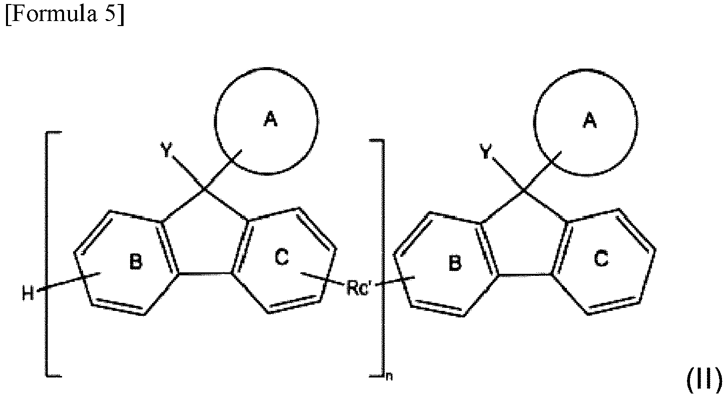 wherein
Ring A represents an aromatic ring; Y is a hydroxyl group, a bromo group, or a chloro group; n represents an integer of 1 to 19; Rc' is a divalent organic group having an aliphatic hydrocarbon group; Rings A, B, and C each independently optionally have at least one selected from an organic group having an aliphatic hydrocarbon group and an electron withdrawing group; when a plurality of Rings A are present, Rings A are the same as or different from each other; when a plurality of Y are present, Y are the same as or different from each other; and when a plurality of Rc' are present, Rc' are the same as or different from each other,
wherein the divalent organic group having an aliphatic hydrocarbon group is a group represented by formula (a): 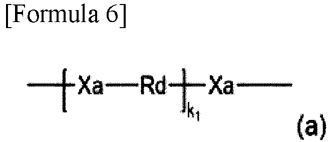 wherein
Xa is absent or represents -O-, -S-, -NHCO-, or -CONH-; Rd represents an aliphatic hydrocarbon group having 5 or more carbon atoms; k₁ represents an integer of 1 to 10; when a plurality of Rd are present, Rd are the same as or different from each other; and when a plurality of Xa are present, Xa are the same as or different from each other, and
wherein the organic group having an aliphatic hydrocarbon group is at least one group selected from the group consisting of
a group represented by formula (b): 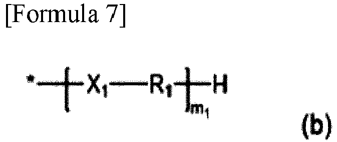 wherein
* represents a binding position; X₁ is -O-; R₁ is an aliphatic hydrocarbon group having 5 to 60 carbon atoms; and m₁ is 1,
a group represented by formula (c): 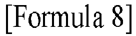 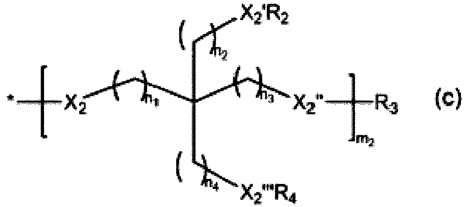 wherein
* represents a binding position; X₂, X₂', X₂", and X₂‴ are -O-; R₂ and R₄ are each independently an aliphatic hydrocarbon group having 5 to 60 carbon atoms; R₃ is an organic group having an aliphatic hydrocarbon group having 5 to 60 carbon atoms; n₁, n₂, n₃, and n₄ are 1; and m₂ is 1, and
a group represented by formula (d): 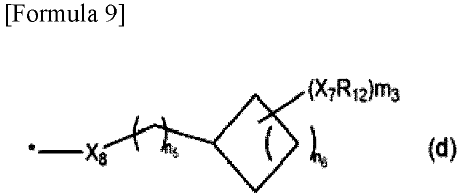 wherein
* represents a binding position; X₈ represents -O-; m₃ is 2 or 3; n₅ is 1; n₆ is 3; X₇ is -O-; m₃ number of R₁₂ are each independently an alkyl group having 4 to 30 carbon atoms,
wherein the organic group having the aliphatic hydrocarbon group is present at the 2- and/or 7-position of the fluorene compound,
(iii) a benzyl compound represented by formula (III): 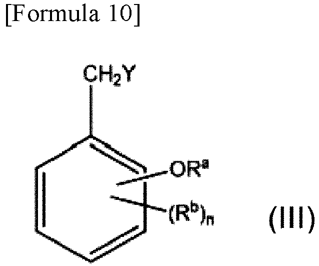 wherein
Y represents a hydroxyl group or an -NHR group, where R represents a hydrogen atom, an alkyl group, or an aralkyl group;
R^{a} is an organic group having an aliphatic hydrocarbon group selected from the group consisting of
a group represented by formula (a): 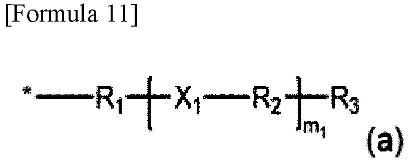 wherein
* represents a binding position; m₁ represents an integer of 1 to 10; m₁ number of X₁ each independently represent a single bond or -O-, -S-, -COO-, -OCONH-, -NHCO-, or -CONH-; R₁ and m₁ number of R₂ each independently represent a divalent aliphatic hydrocarbon group having 5 or more carbon atoms; and R₃ is a hydrogen atom, or
a group represented by formula (III'): 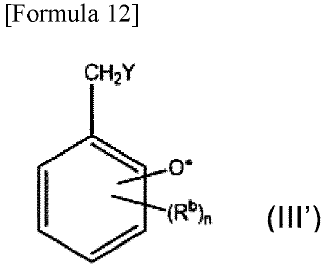 wherein
Y represents a hydroxyl group or an -NHR group, where R represents a hydrogen atom, an alkyl group, or an aralkyl group; * represents a binding position; n number of R^{b} each independently represent an alkoxy group having 1 to 6 carbon atoms, a halogen atom, or an alkyl group having 1 to 6 carbon atoms optionally substituted with one or more halogen atoms; and n represents an integer of 0 to 4;
a group represented by formula (b): 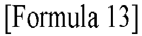 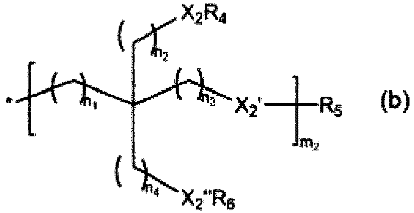 wherein
* represents a binding position; m₂ represents 1 or 2; n₁, n₂, n₃, and n₄ each independently represent an integer of 0 to 2; m₂ number of X₂, m₂ number of X₂', and m₂ number of X₂" each independently represent a single bond or -O-, -S-, -COO-, -OCONH-, -NHCO-, or -CONH-; m₂ number of R₄ and m₂ number of R₆ each independently represent an aliphatic hydrocarbon group having 5 or more carbon atoms; and R₅ represents an aliphatic hydrocarbon group having 5 or more carbon atoms;
a group represented by formula (c): 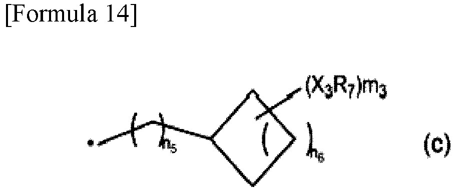 wherein
* represents a binding position; m₃ represents an integer of 0 to 15; n₅ represents an integer of 0 to 11; n₆ represents an integer of 0 to 5; m₃ number of X₃ each independently represent a single bond or -O-, -S-, -COO-, -OCONH-, -NHCO-, or -CONH-; and m₃ number of R₇ each independently represent a hydrogen atom, a methyl group, or an aliphatic hydrocarbon group having 5 or more carbon atoms; and
a group represented by formula (d): 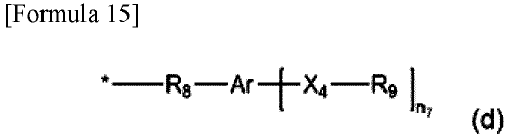 wherein
* represents a binding position; n₇ number of X₄ each independently represent a single bond or -O-, -S-, -COO-, -OCONH-, -NHCO-, or -CONH-; R₈ represents a divalent aliphatic hydrocarbon group; n₇ number of R₉ each independently represent a monovalent aliphatic hydrocarbon group; n₇ represents an integer of 1 to 5; and Ar represents an arylene group,
the organic group having a total number of carbon atoms of 30 or more;
n number of R^{b} each independently represent an alkoxy group having 1 to 6 carbon atoms, a halogen atom, or an alkyl group having 1 to 6 carbon atoms optionally substituted with one or more halogen atoms; and n represents an integer of 0 to 4,
(iv) a compound represented by formula (IV): 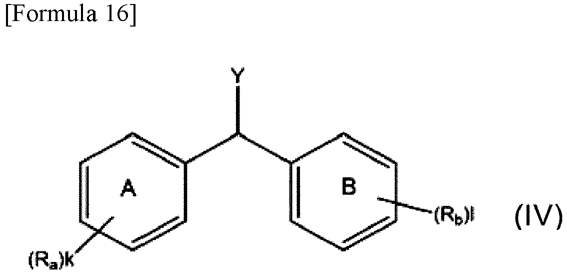 wherein
Y represents a hydroxyl group or an -NHR group, where R represents a hydrogen atom, an alkyl group, or an aralkyl group; k and l each independently represent an integer of 0 to 5, provided that k+1 is not 0; k number of Rₐ and one R^{b} each independently represent an organic group having an aliphatic hydrocarbon group having 5 or more carbon atoms selected from the group consisting of
a group represented by formula (a): 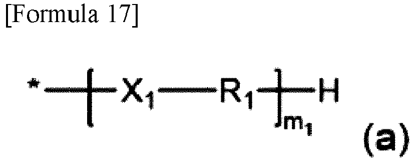 wherein
* represents a binding position; m₁ represents an integer of 1 to 10; m₁ number of X₁ each independently are absent or represent -O-, -S-, -COO-, -OCONH-, or -CONH-; m₁ number of R₁ each independently represent a divalent aliphatic hydrocarbon group having 5 or more carbon atoms,
a group represented by formula (b): 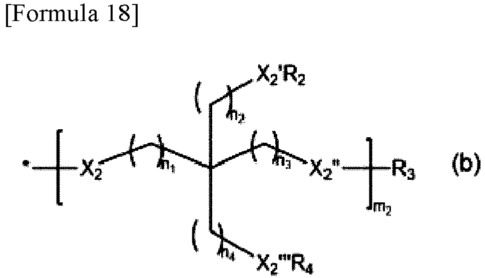 wherein
* represents a binding position; m₂ represents an integer of 1 or 2; m₂ number of n₁, n₂, n₃, and n₄ each independently represent an integer of 0 to 2; m₂ number of X₂, m₂ number of X₂', m₂ number of X₂‴, and m₂ number of X₂" each independently are absent or represent -O-, -S-, -COO-, -OCONH-, or -CONH-; m₂ number of R₂ and R₄ each independently represent a hydrogen atom, a methyl group, or an aliphatic hydrocarbon group having 5 or more carbon atoms; and R₃ represents an aliphatic hydrocarbon group having 5 or more carbon atoms, and
a group represented by formula (e): 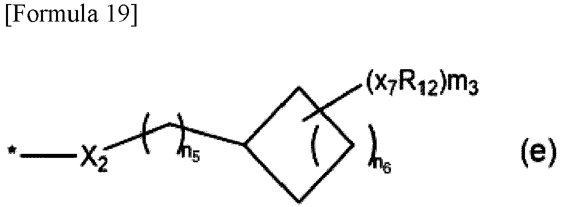 wherein
* represents a binding position; m₃ represents an integer of 0 to 15; n₅ represents an integer of 0 to 11; n₆ represents an integer of 0 to 5; X₂ is absent or represents -O-, -S-, -NHCO-, or - CONH-; m₃ number of X₇ each independently are absent or represent -O-, -S-, -COO-, -OCONH-, -NHCO-, or -CONH-; and m₃ number of R₁₂ each independently represent a hydrogen atom, a methyl group, or an aliphatic hydrocarbon group having 5 or more carbon atoms,
wherein the total number of carbon atoms of all aliphatic hydrocarbon groups in an organic group having k+1 number of aliphatic hydrocarbon groups is 16 or more; Ring A optionally further has a substituent in addition to Rₐ; and Ring B optionally further has a substituent in addition to R_{b},
(v) a branched chain-containing aromatic compound represented by formula (V): 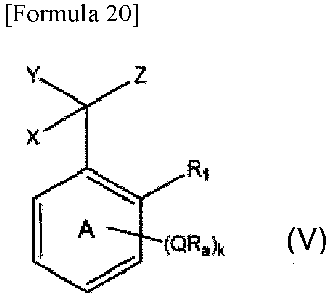 wherein
k number of Q each independently represent a single bond or -O-, -S-, -C(=O)O-, - C(=O)NH-, or -NH-; k number of Rₐ each independently represent an organic group having at least one aliphatic hydrocarbon group having one or more branched chains with a total number of branched chains of 3 or more and a total number of carbon atoms of 14 or more and 300 or less; k represents an integer of 1 to 4; R₁ is a hydrogen atom or, when Z is a group represented by formula (a) below, represents a single bond together with R₂ to form a fluorene ring together with Ring B; Ring A optionally has at least one substituent selected from the group consisting of a halogen atom, a C1-6 alkyl group optionally substituted with one or more halogen atoms, and a C1-6 alkoxy group optionally substituted with one or more halogen atoms, in addition to R₁, k number of QRa, and C(X)(Y)Z; X represents a hydrogen atom or a phenyl group; Y represents a hydroxyl group or an -NHR group, where R represents a hydrogen atom, an alkyl group or an aralkyl group; and Z represents a hydrogen atom or
a group represented by formula (a): 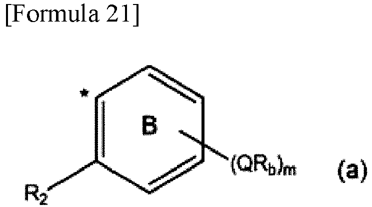
wherein
* represents a binding position; m represents an integer of 0 to 4; m number of Q each independently represent a single bond or -O-, -S-, -C(=O)O-, -C(=O)NH-, or -NH-; m number of R_{b} each independently represent an organic group having at least one aliphatic hydrocarbon group having one or more branched chains with a total number of branched chains of 3 or more and a total number of carbon atoms of 14 or more and 300 or less; R₂ represents a hydrogen atom or represents a single bond together with R₁ to form a fluorene ring together with Ring A; and Ring B optionally has one or more substituents selected from the group consisting of a halogen atom, a C1-6 alkyl group optionally substituted with one or more halogen atoms, and a C1-6 alkoxy group optionally substituted with one or more halogen atoms, in addition to m QR_{b}s and R₂; and
the organic group having at least one aliphatic hydrocarbon group having one or more branched chains with a total number of branched chains of 3 or more and a total number of carbon atoms of 14 or more and 300 or less in Rₐ and R_{b} is a group having 3 or more same or different divalent groups represented by formula (b): 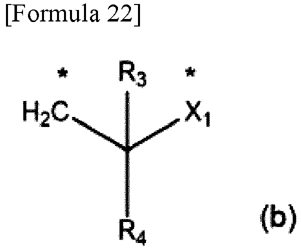 wherein
* represents a binding position to an adjacent atom; R₃ and R₄ each independently represent a hydrogen atom or a C1-4 alkyl group; and X₁ represents a single bond, a C1-4 alkylene group, or an oxygen atom, provided that R₃ and R₄ are not simultaneously hydrogen atoms,
(vi) a compound represented by formula (VI): 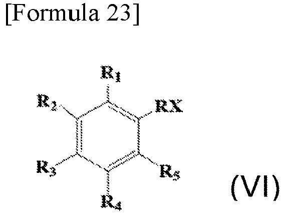 wherein
R₁ and R₅ are each a hydrogen atom; R₂, R₃, and R₄ are each an alkoxy group having 8 to 30 carbon atoms; RX is a group binding to a carboxyl group, an amino group, or a hydroxy group of an amino acid, a peptide, or an amino acid amide or to a linker, which is represented by -CHO, -CHzOH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group,
(vii) a compound represented by formula (VII): 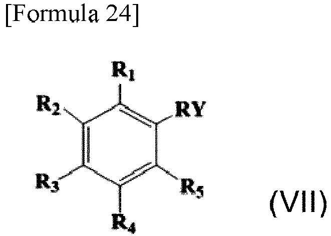 wherein
R₂, R₄, and R₅ are each a hydrogen atom; R₁ and R₃ are each an alkoxy group having 12 to 30 carbon atoms; RY is a group binding to a carboxyl group, an amino group, or a hydroxy group of an amino acid, a peptide, or an amino acid amide or to a linker, which is represented by -CHO, -CHzOH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group,
(viii) a compound represented by formula (VIII): 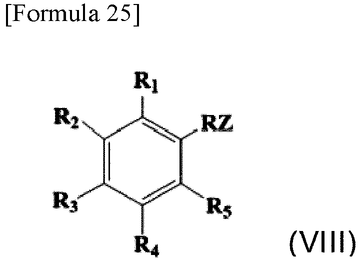 wherein
R₁, R₃, and R₅ are each a hydrogen atom; R₂ and R₄ are each an alkoxy group having 12 to 30 carbon atoms; RZ is a group binding to a carboxyl group, an amino group, or a hydroxy group of an amino acid, a peptide, or an amino acid amide or to a linker, which is represented by -CHO, -CHzOH, -CHR₆-NHR₇, or CH₂SH, where R₇ represents a hydrogen atom, an alkyl group having 1 to 6 carbon atoms, a benzyl group, or an alkoxy-substituted benzyl group; and R₆ represents a hydrogen atom, a phenyl group, or an alkoxy-substituted phenyl group, and
(ix) a compound represented by formula (IX): 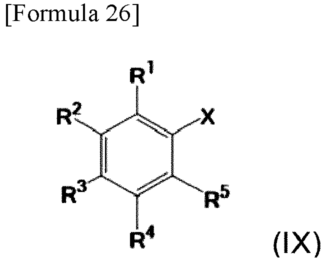 wherein
X is a group representing -CHzORa where Ra represents a hydrogen atom, a halogenocarbonyl group, or an active ester protecting group, -CH₂NHRb where Rb represents a hydrogen atom, a linear or branched alkyl group having 1 to 6 carbon atoms, or an aralkyl group, a halogenomethyl group, a methyl azide group, a formyl group, or an oxime, and binding to a carboxyl group, an amino group, or a hydroxy group of an amino acid, a peptide, or an amino acid amide or to a linker; at least one of R¹, R², R³, R⁴ and R⁵ represents a group represented by a formula of -O-R⁶-Xa-A, and a residual group represents a hydrogen atom, a halogen atom, an alkyl group having 1 to 4 carbon atoms, or an alkoxy group having 1 to 4 carbon atoms; R⁶ represents a linear or branched alkylene group having 1 to 16 carbon atoms, and Xa represents O or CONRc where Rc represents a hydrogen atom or an alkyl group having 1 to 4 carbon atoms; and A represents any one of
formulae (1) to (11) 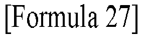 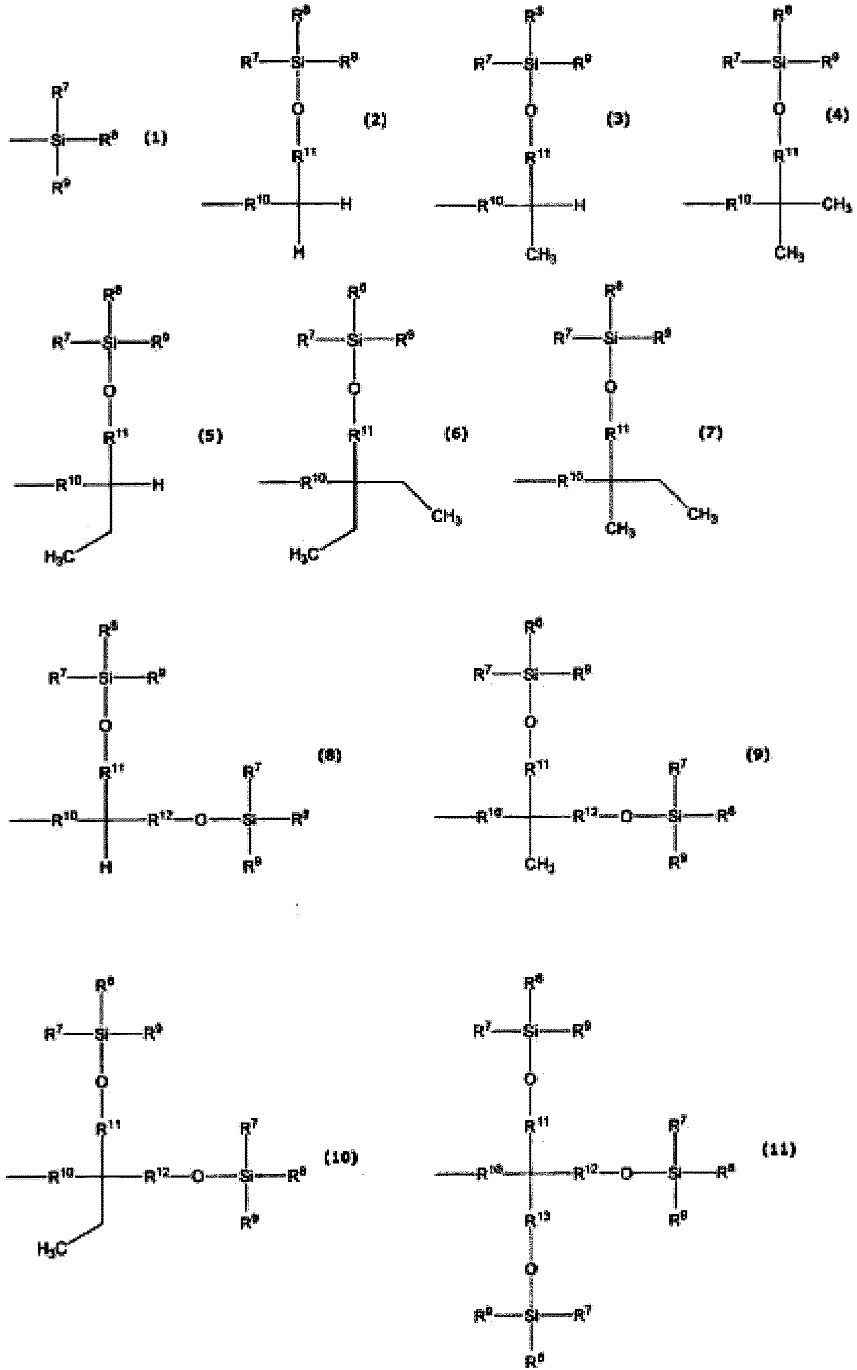
wherein R⁷, R⁸, and R⁹ are the same as or different from each other and represent a linear or branched alkyl group having 1 to 6 carbon atoms or an aryl group optionally having a substituent, R¹⁰ represents a single bond or a linear or branched alkylene group having 1 to 3 carbon atoms, and R¹¹, R¹², and R¹³ are the same as or different from each other and represent a linear or branched alkylene group having 1 to 3 carbon atoms.

4. The method according to claim 1, wherein the N-terminal protecting group is selected from the group consisting of a 9-fluorenylmethyloxycarbonyl group (Fmoc group), a *tert*-butoxycarbonyl group (Boc group), a benzyloxycarbonyl group (Cbz group), and an allyloxycarbonyl group (Alloc group).

5. The method according to claim 1, further comprising, after the condensation step,
a capping step of introducing a reaction mixture containing the N-terminally and C-terminally protected peptide extended N-terminally and a capping agent having an amino group into a flow reactor, thereby reacting an unreacted C-terminally activated derivative of the N-terminally protected amino acid or peptide with the capping agent in the flow reactor to inactivate the C-terminally activated derivative, preferably wherein the capping agent having an amino group is a compound selected from the group consisting of 2-(2-aminoethoxyethanol) (AEE) and 2-aminoethyl hydrogen sulfate (AEHS).

6. An apparatus for producing a peptide, comprising:
a C-terminal activation unit comprising a flow reactor and a raw material introducing member for introducing an N-terminally protected amino acid or peptide, in which an N-terminal amino group is protected by an N-terminal protecting group, and a C-terminal activator into the flow reactor, for activating a C-terminal carboxyl group of the N-terminally protected amino acid or peptide in the flow reactor to form a C-terminally activated derivative of the N-terminally protected amino acid or peptide;
a condensation unit comprising a flow reactor and a raw material introducing member for introducing the C-terminally activated derivative of the N-terminally protected amino acid or peptide and a C-terminally protected amino acid or peptide, in which a C-terminal carboxyl group is protected by a tag protecting group, into the flow reactor, for condensing the N-terminally protected amino acid or peptide with the C-terminally protected amino acid or peptide on the N-terminal side in the flow reactor to form an N-terminally and C-terminally protected peptide extended N-terminally;
an N-terminal deprotection unit comprising a flow reactor and a raw material introducing member for introducing the N-terminally and C-terminally protected peptide extended N-terminally and a deprotecting agent for an N-terminal protecting group into the flow reactor, for removing the N-terminal protecting group for deprotection in the flow reactor to form a C-terminally protected peptide extended N-terminally; and
an extraction unit comprising a mixing member for mixing a reaction mixture containing the C-terminally protected peptide extended N-terminally with an aqueous phase, for mixing the reaction mixture containing the C-terminally protected peptide extended N-terminally with the aqueous phase to extract the C-terminally protected peptide extended N-terminally into an organic phase.

7. The apparatus according to claim 6, further comprising
a capping unit comprising a flow reactor and a raw material introducing member for introducing a reaction mixture containing the N-terminally and C-terminally protected peptide extended N-terminally and a capping agent having an amino group into the flow reactor, for reacting an unreacted C-terminally activated derivative of the N-terminally protected amino acid or peptide with the capping agent in the flow reactor to inactivate the C-terminally activated derivative.

8. The apparatus according to claim 6, wherein the C-terminal activation unit, the condensation unit, and the N-terminal deprotection unit each comprises two or more inlets, a mixer for mixing a reaction liquid flowing in from the two or more inlets, one or more outlets for allowing the reaction liquid mixed in the mixer to flow out, a pump for feeding a raw material, and a temperature control device for controlling temperatures of the inlets, the outlets, the mixer, and a flow channel of the pump.

9. The apparatus according to claim 6, wherein the extraction unit comprises two or more inlets, a mixer for mixing a reaction liquid containing the C-terminally protected peptide extended N-terminally flowing in from the two or more inlets with the aqueous phase, a phase separating member for separating a mixed phase of the reaction liquid containing the C-terminally protected peptide extended N-terminally and the aqueous phase, which are mixed in the mixer, into an organic phase and an aqueous phase, one or more outlets for discharging the organic phase separated by the phase separating member, and a pump for feeding the aqueous phase, preferably wherein the extraction unit comprises at least one physical property detecting member selected from the group consisting of a pressure gauge, a flowmeter, and a thermometer in the middle of the inlet and/or the outlet.

10. The apparatus according to claim 8, wherein at least one of the C-terminal activation unit, the condensation unit, and the N-terminal deprotection unit comprise at least one physical property detecting member selected from the group consisting of a pressure gauge, a flowmeter, and a thermometer in the middle of the inlet and/or the outlet.

11. The apparatus according to claim 6, wherein at least one of the C-terminal activation unit, the condensation unit, the N-terminal deprotection unit, and the extraction unit comprises at least one compound detecting member for detecting a compound contained in the raw material and the reaction liquid.

12. The apparatus according to claim 11, wherein the at least one compound detecting member is at least one member selected from the group consisting of an ultraviolet-visible spectroscopy detector, a photodiode array detector, a fluorescence detector, a differential refractive index detector, an electrical conductivity detector, an evaporative light scattering detector, an infrared spectrometer, a near-infrared spectrometer, a nuclear magnetic resonance device, and a mass spectrometer.

13. The apparatus according to claim 11, further comprising a compound separating member for separating a compound to be detected by the compound detecting member at a front part of the compound detecting member in a flow channel of, for example, the raw material and the reaction liquid.

14. The apparatus according to claim 6, wherein at least one of the C-terminal activation unit, the condensation unit, the N-terminal deprotection unit, and the extraction unit further comprises at least one outlet port for sampling the reaction liquid.

15. The apparatus according to claim 6, further comprising a circulating flow unit for circulating and flowing the C-terminally protected peptide extended N-terminally obtained in the extraction unit into the raw material introducing member in the condensation step.
